# EUROPÄISCHE PATENTSCHRIFT

(11) **EP 0 907 633 B1**
(45) Veröffentlichungstag und Bekanntmachung des Hinweises auf die Patenterteilung: **22.08.2001**
(21) Anmeldenummer: 97923887.0
(22) Anmeldetag: 14.05.1997
(51) Int. Cl.: C07C 251/16, C07C 251/24, C07C 245/08, C07C 235/64, C07C 251/86, C07D 501/34, C07D 499/00, C07D 501/46, C07D 501/24

(54) **NEUE SYNTHETISCHE CATECHOLDERIVATE, VERFAHREN ZU IHRER HERSTELLUNG UND IHRE VERWENDUNG**
NEW SYNTHETIC CATECHOL DERIVATIVES, METHOD FOR PRODUCTION AND USE THEREOF
NOUVEAUX DERIVES SYNTHETIQUES DE CATECHOL, LEUR PROCEDE DE PRODUCTION ET LEUR UTILISATION

(30) Priorität: 26.06.1996 DE 19625524
(43) Veröffentlichungstag der Anmeldung: 14.04.1999
(73) Patentinhaber: Grünenthal GmbH, 52078 Aachen (DE)
(72) Erfinder: HEINISCH, Lothar, D-07743 Jena (DE); MÖLLMANN, Ute, D-07749 Jena (DE); SCHNABELRAUCH, Matthias, D-07745 Jena (DE); REISSBRODT, Rolf, D-38855 Wernigerode (DE)
(86) Internationale Anmeldenummer: EP9702453
(87) Internationale Veröffentlichungsnummer: WO9749670

(56) Entgegenhaltungen:
- EP-A- 0 189 287
- EP-A- 0 267 733
- EP-A- 0 341 948
- EP-A- 0 472 062
- DE-A- 3 414 049
- CHEMICAL ABSTRACTS, vol. 120, no. 19, 9.Mai 1994 Columbus, Ohio, US; abstract no. 244406z, XP002037932 & ZHONGHUA YAOXUE ZAZHI, Bd. 45, Nr. 6, 1993, Seiten 563-572, T.L. TSOU ET AL:
- CHEMICAL ABSTRACTS, vol. 117, no. 23, 7.Dezember 1992 Columbus, Ohio, US; abstract no. 229985g, XP002037933 & ANTIMICROB. AGENTS CHEMOTHER., Bd. 36, Nr. 10, 1992, Seiten 2166-2175, A. BROCHU ET AL:
- CHEMICAL ABSTRACTS, vol. 117, no. 10, 7.September 1992 Columbus, Ohio, US; abstract no. 98611g, XP002037934 & POLYHEDRON, Bd. 11, Nr. 10, 1992, Seiten 1161-1168, M. APLINCOURT ET AL:
- CHEMICAL ABSTRACTS, vol. 117, no. 9, 31.August 1992 Columbus, Ohio, US; abstract no. 90739j, XP002037935 & J. AM. CHEM. SOC., Bd. 114, Nr. 17, 1992, Seiten 6661-6671, Y. TOR ET AL:
- CHEMICAL ABSTRACTS, vol. 115, no. 21, 25.November 1991 Columbus, Ohio, US; abstract no. 222814a, XP002037936 & BIOL. MET., Bd. 4, Nr. 1, 1991, Seiten 62-69, M.J. MILLER ET AL:
- CHEMICAL ABSTRACTS, vol. 108, no. 11, 14.März 1988 Columbus, Ohio, US; abstract no. 94247j, XP002037937 & CHEM. PHARM. BULL., Bd. 35, Nr. 5, 1987, Seiten 1903-1909, N. OHI ET AL:

## Beschreibung

Die vorliegende Erfindung betrifft neue synthetische Catecholderivate, bei denen aromatische Azomethincarbonsäuren, Benzhydrazone, Aminosäuren, Aminobenzoesäuren oder Dipeptide, Pyrrolidin- und/oder Oxazolidincarbonsäuren oder Formylcarboxymethyloxime als Strukturelemente fungieren, sowie deren Konjugate mit Wirkstoffen, insbesondere Antibiotika.

Es ist bekannt, daß bestimmte Catecholstrukturen in natürlichen Siderophoren als eisenkomplexierende Strukturelemente eine wesentliche Rolle spielen ("Iron Transport in Microbes, Plants and Animals", Hrsg.: Winkelmann, G., van Helm, D., Neilands, J. B., V.Ch.-Verlagsgesellschaft Weinheim, 1987), z. B. ist das Enterobactin, ein Siderophor bei *E. coli* und anderen Bakterienstämmen, ein Trimeres aus N-(2,3-Dihydroxybenzoyl)-L-serin. Auch das Monomer ist als Siderophor wirksam (Hantke, K., FEMS Microbiol. Lett. 67 (1990), 5). Das N-(2,3-Dihydroxybenzoyl)glycin ist als Siderophor bei *B*. *subtilis* gefunden worden (Ito, T., Neilands, J. B., J. Amer. Chem. Soc. 80 (1958), 4645). Einige catecholsubstituierte Aminosäurederivate sind bereits synthetisch hergestellt worden, z. B. das N-(2,3-Dihydroxybenzoyl)-L-threonin (Kanai, F. Kaneko, T., Morishima, H., Isshiki, K., Taketa. T., Takeuchi, T., Umezawa, H., J. Antibiot. 38 (1985), 39), das N²,N⁶-Bis-(2,3-dihydroxybenzoyl)-L-lysin (Corbin, J. L., Bulen, W. A., Biochemistry 8 (1969), 757; McKee, J. A., Sharma, S. K., Miller, M. J.; Bioconjugate Chem. 2 (1991) 281) und N²,N⁶-Bis-(2,3-dihydroxybenzoyl)-Iysyl-N⁶-(2,3-dihydroxybenzoyl)lysin (Chimiak, A., Neilands, J. B. Structure and Bonding 58, (1984), 89). Weiterhin ist bekannt, daß bei verschiedenen Bakterienstämmen bestimmte Glyoxylsäurebenzhydrazone, Oxanilsäurederivate u. a. als Siderophore dienen können (Reissbrodt, R., Heinisch, L., Möllmann, U., Rabsch, W., Ulbricht, H., Bio. Metals 6 (1993), 155). Einige Dihydroxybenzylidenaminobenzoesäuren sind bereits in der Literatur beschrieben, jedoch ohne Angaben einer Siderophorwirksamkeit (Takita, H., Noda, S., Inada, K., Mukaida, Y. S., Toji, M. K., Kobayashi, H., DE 3 414 049 (1984), H. Wolf, Monatsh. Chem. 31 (19101, 903).

Obwohl verschiedene Catecholverbindungen mit β-Lactamen verknüpft wurden, mit denen eine Steigerung der antibakteriellen Wirksamkeit dieser Antibiotika, bedingt durch eine Einschleusung über bakterielle Eisentransportwege in die Bakterienzelle, erzielt wurde, (z. B. Arisawa, M., Sekine, Y., Shimizu, S., Takano, H., Angehrn, P., Then, R. L., Antimicrob. Agents Chemother. 35, (1991), 653) besteht ein großer Bedarf nach weiteren neuen synthetischen Siderophoren mit verbesserten pharmakologischen und pharmakokinetischen Eigenschaften, die zur Konjugatbildung mit Antibiotika geeignet sind.

Siderophore sind andererseits als Eisenchelatoren potentiell in der Lage, den biologischen Eisenstoffwechsel sowie damit zusammenhängende Erkrankungen in verschiedener Weise zu beeinflussen. So wird das Siderophor Desferrioxamin (Desferal) erfolgreich bei Erkrankungen, die auf Eisenüberladung beruhen (z. B. Thalassämie) eingesetzt.

Der Erfindung liegt die Aufgabe zugrunde, neue synthetische Catecholderivate mit aromatischen Azomethincarbonsäuren, Benzhydrazonen, Aminosäuren, Aminobenzoesäuren bzw. Dipeptiden, Pyrrolidin- bzw. Oxazolidincarbonsäuren sowie Formylcarboxymethyloxime als Basisstrukturen aufzufinden, die als Siderophore und/oder biologische Eisenchelatoren fungieren können und die in Form ihrer Konjugate mit Wirkstoffen, insbesondere Antibiotika, eine verbesserte Penetration dieser Verbindungen in Bakterienzellen bewirken und damit deren antibakterielle Wirksamkeit erhöhen sowie eine bessere Bekämpfung penetrationsbezogener Antibiotikaresistenz bei bakteriellen Infektionen ermöglichen.

Die erfindungsgemäßen Verbindungen sind als Siderophore bei gramnegativen Bakterien wirksam, d. h. sie können Bakterien mit Eisenionen versorgen und in Form ihrer Konjugate mit Wirkstoffen, insbesondere mit Antibiotika (als "Siderophor-Antibiotikakonjugate") diese über Eisentransportwege in die Bakterienzelle einschleusen und somit deren Wirksamkeit verbessern und sogar noch erweitern. Diese Verbindungen können insbesondere zur Bekämpfung penetrationsbezogener Antibiotikaresistenz eingesetzt werden. Außerdem stellen die erfindungsgemäßen Verbindungen Eisenchelatoren dar und können den biologischen Eisenstoffwechsel und damit zusammenhängende Erkrankungen in verschiedener Weise beeinflussen.

Darüber hinaus sind die erfindungsgemäßen Verbindungen besser wirksam und einfacher herstellbar als bisher bekannte Verbindungen und ermöglichen in Form ihrer Konjugate mit Wirkstoffen eine bessere Bekämpfung penetrationsbezogener Antibiotikaresistenz bei bakteriellen Infektionen. Weiterhin werden mit dieser Erfindung neue Eisenchelatoren bereitgestellt, die in verschiedener Weise den biologischen Eisenstoffwechsel und damit zusammenhängende Erkrankungen beeinflussen können.

Es werden neue synthetische Catecholderivate der allgemeinen Formel I bereitgestellt, 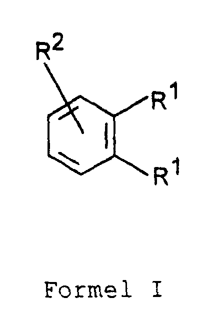 in der R¹ identisch oder unabhängig voneinander OH und/oder OAcyl bedeutet und R² in 3- und/oder 4-Stellung folgende Gruppen darstellt:
a. aromatische Azomethincarbonsäurereste und/oder Azobenzolcarbonsäurereste: 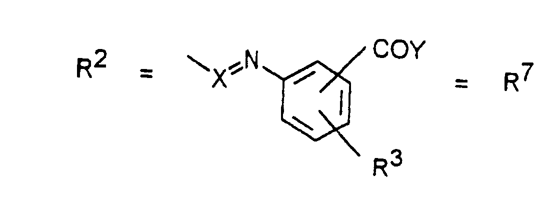
   - X =: CH, N, CH=CH-CH
   - Y =: OA, mit A = H, Alkyl, Aryl, Aralkyl, ein Alkalimetallion (vorzugsweise Na, K), ein Ammoniuminon oder ein substituiertes Ammoniumion ist, oder
   - Y =: ein Wirkstoffrest, der eine OH- oder NH-Gruppe enthält, ist, ausgewählt aus der Antibiotikagruppe, die die β-Lactame Cephalosporin, insbesondere Cephalexin, Cephadroxil oder Claforan, und Penicillin, insbesondere Ampicillin oder Amoxicillin, und ferner Tetracyclinderivate vom Typ des Aminodoxycyclins sowie Antibiotika vom Typ der Aminoglykoside, Makrolide, Chinolone und Carbapeneme umfaßt,

   - R³ =: ein oder zwei OAcyl-Reste, wenn R¹ = OH oder OAcyl oder
   - R³ =: H, wenn R¹ = OAcyl oder 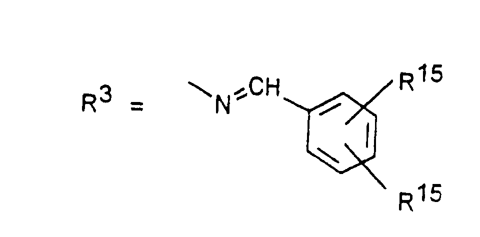
   - R¹⁵: identisch oder unabhängig voneinander H, OAcyl bedeutet oder 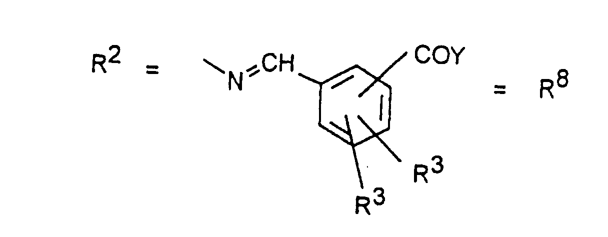
   Y = OA mit A = H, Alkyl, Aryl, Aralkyl, ein Alkalimetallion (insbesondere Na, K), ein Ammoniumion oder ein substituiertes Ammoniumion, oder
   Y = ein Wirkstoffrest, der eine NH- oder OH-Gruppe enthält, ist, wie oben definiert,
   R³ identisch oder unabhängig voneinander H, OH, Oacyl bedeutet
b. Benzhydrazonreste: 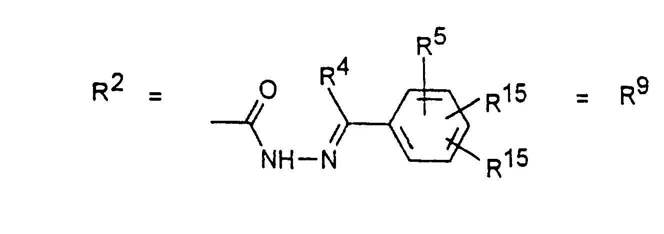
   - R¹⁵: identisch oder unabhängig voneinander H, OH, OAcyl bedeutet,
   - R⁴ und/oder R⁵ =: H, COY, wobei
   - Y =: OA mit A = H, Alkyl, Aryl, Aralkyl, ein Alkalimetallion (vorzugsweise Na, K) ein Ammoniumion oder ein substituiertes Ammoniumion oder
   - Y =: ein Wirkstoffrest, der eine NH- oder OH-Gruppe enthält, ist, wie oben definiert,
c. Aminobenzoesäurereste: 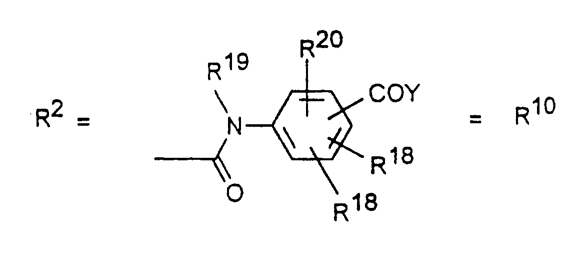
   - Y =: OA mit A = H, Alkyl, Aryl, Aralkyl, ein Alkalimetallion (vorzugsweise Na, K), ein Ammoniumion oder ein substituiertes Ammoniumion, oder
   - Y =: ein Wirkstoffrest, der eine NH- oder OH-Gruppe enthält, ist, wie oben definiert,
   - R¹⁹ =: H, Alkyl
   - R²⁰ =: H, Alkyl, Halogen, OH, OAlkyl, OAcyl oder 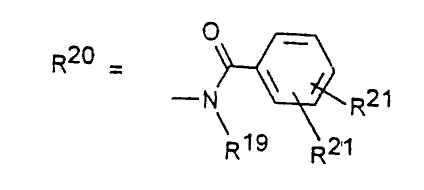
   - R¹⁸ und R²¹: jeweils identisch oder unabhängig voneinander H, OH, OAcyl, OAlkyl in 2,3- oder 3,4-Position bedeuten,
d. Aminosäurereste: 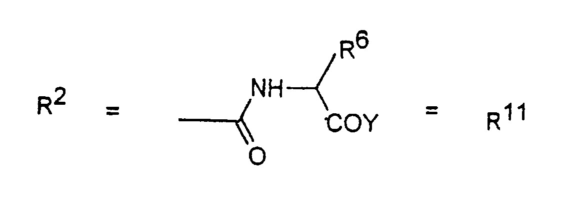
   - Y =: OA mit A = H, Alkyl, Aryl, Aralkyl, ein Alkalimetallion (insbesondere Na, K), ein Ammoniumion oder ein substituiertes Ammoniumion, oder
   - Y =: ein Wirkstoffrest, der eine NH- oder OH-Gruppe enthält, ist, wie oben definiert,
   - R⁶ =: Alkyl, Hydroxyalkyl (mit C₁ - C₅, wenn R¹ = OAcyl, und C₃ - C₅, wenn R¹ = OH), Alkoxyalkyl, Acyloxyalkyl, Arylalkoxyalkyl, oder 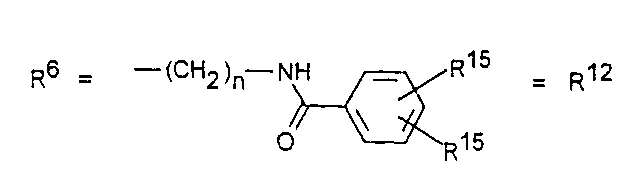
   - R¹⁵: identisch oder unabhängig voneinander H, OH, Oacyl bedeutet,
   - n: eine ganze Zahl zwischen 1 und 5 ist, wenn R¹ Oacyl und R¹⁵ H und/oder Oacylist oder
   - n: eine ganze Zahl zwischen 1 und 3 ist, wenn R¹ OH und R¹⁵ H und/oder OH ist, oder 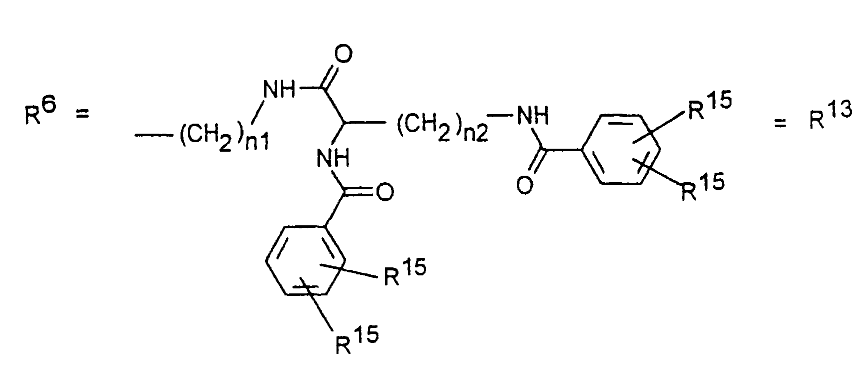
   - R¹⁵: identisch oder unabhängig voneinander H, OH, OAcyl bedeutet, n₁ und n₂ eine ganze Zahl zwischen 1 und 5 ist
e. Pyrrolidin- und/oder Oxazolidincarbonsäurereste: 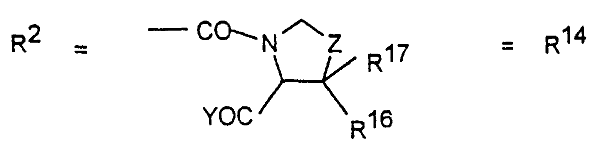
   - Z =: O, CH₂
   - R¹⁶ und R¹⁷: identisch oder unabhängig voneinander H, Alkyl oder Aryl bedeuten
   - Y =: OA mit A = H, Alkyl, Aryl, Aralkyl, ein Alkalimetallion (vorzugsweise Na, K), ein Ammonium oder ein substituiertes Ammoniumion, oder
   - Y =: ein Wirkstoffrest, der eine NH- oder OH-Gruppe enthält, ist,wie oben definiert,
f. Formyl-O-carboxymethyloxime:
   - R² =: CH=NOCH₂COY, wobei
   Y = OA, mit A = H, Alkyl, Aryl, Aralkyl, ein Alkalimetallion (vorzugsweise Na, K), ein Ammoniumion oder ein substituiertes Ammoniumion, oder
   Y = ein Wirkstoffrest, der eine NH- oder OH-Gruppe enthält, ist, wie oben definiert.

In den vorstehenden Formeln und im folgenden bedeuten Acyl insbesondere geradkettiges oder verzweigtes C₁ - C₆ Alkanoyl oder geradkettiges oder verzweigtes C₁ - C₆-Alkoxy-carbonyl, geradkettiges oder verzweigtes Alkyl und geradkettiges oder verzweigtes Alkoxy, auch in Wortkombinationen wie geradkettiges oder verzweigtes Alkoxyalkyl oder Acyloxyalkyl, sind insbesondere geradkettiges oder verzweigtes C₁ - C₈-Alkyl oder. -Alkoxy, Aryl, insbesondere Phenyl und substituiertes Phenyl, wie durch geradkettiges oder verzweigtes Alkyl, Halogen, insbesondere Cl, F, geradkettiges oder verzweigtes Alkoxy, Hydroxy, Carboxy, geradkettiges oder verzweigtes Alkoxycarbonyl und halogensubstituiertes Alkyl, substituiertes Phenyl, und Aralkyl besonders Phenylmethyl und 1- oder 2-Phenylethyl. Die angegebenen Reste R³, R⁵, R¹⁵, R²⁰ und COY können in allen möglichen Positionen stehen. Ein substituiertes Ammoniumion ist z. B. ein durch Alkyl substituiertes Ammoniumion.

Ein Wirkstoffrest bedeutet den Rest eines geeigneten antibakteriellen Wirkstoffes mit freier NH- oder OH-Gruppe, wobei der Wirkstoff über diese NH- oder OH-Gruppe mit dem Catecholrest verestert bzw. amidiert ist. Die Bindung zwischen dem Catecholderivat und dem Antibiotikum kann sowohl direkt als auch über gebräuchliche Linkergruppen, z. B. Aminocarbonsäuren, Hydroxycarbonsäuren, Diamine oder Diole erfolgen. Unter einem Antibiotikum ist ein β-Lactam Cephalosporin, z. B. Cephalexin, Cephadroxil oder Claforan, oder ein Penicillin, z. B. Ampicillin, Amoxicillin, oder ein Tetracyclinderivat, z. B. ein Aminodoxycyclin, oder ein Antibiotikum vom Typ der Aminoglykoside, Makrolide, Chinolone oder Carbapeneme zu verstehen.

Im Falle des Vorliegens asymmetrischer C-Atome sind die entsprechenden D- und L-Formen, Enantiomere und Diastereomere sowie die Racemate bzw. Enantiomeren- und Diastereomerengemische ebenfalls Gegenstand der Erfindung.

Die erfindungsgemäßen Verbindungen werden z. B. hergestellt, indem
a. catecholsubstituierte Benzaldehyde (Formel I mit R² = CHO) in einem geeigneten Lösungsmittel, wie Ethanol oder Toluol mit einem Wasserabscheider oder mit wasserbindenden Mitteln, wie Molekularsieb, in einem Soxhletaufsatz, bei Reaktionstemperaturen zwischen +50° C und +120° C, im allgemeinen bei der Siedetemperatur des Lösungsmittels, mit entsprechenden Aminobenzoesäuren zu aromatischen Azomethincarbonsäuren (Formel I mit R² = R⁷ oder R⁸) umgesetzt werden,
   oder indem
b. catecholsubstituierte Benzhydrazide (Formel I mit R² = CONHNH₂ in einem geeigneten Lösungsmittel, wie Wasser, Ethanol oder Essigsäure, bei Temperaturen zwischen +10° C und +120° C, vorzugsweise bei der Siedetemperatur des Lösungsmittels, mit entsprechenden substituierten Formylbenzoesäuren bzw. mit Phenylglyoxylsäuren zu entsprechenden Benzhydrazonen (Formel I mit R² = R⁹) umgesetzt werden,
   oder indem
c. Di (acyloxy)benzoylchloride (Formel I z. B. mit R¹ = OCOCH₃ und R²=COCI) mit Aminobenzoesäuren oder deren Estern in einem geeigneten Lösungsmittel, wie Tetrahydrofuran zusammen mit einem tertiären Amin, z. B. Triethylamin, bei einer Temperatur zwischen - 30° C und +20° C, oder in wäßriger Natriumbicarbonatlösung bei 0° C bis +10° C zu N-[Di(acyloxy)benzoyl] aminobenzoesäuren bzw. -estern umgesetzt werden und letztgenannte Ester gegebenenfalls in die freien Säuren (Formel I mit R² = R¹⁰) umgewandelt werden,
   oder indem
d. 2,3-(Di(benzyloxy)benzoylchlorid (Formel I mit R¹ = OCH₂C₆H₅ und R² = COCI) in einem geeigneten Lösungsmittel wie Tetrahydrofuran zusammen mit einem tertiären Amin, z. B. Triethylamin, bei einer Temperatur zwischen -30° C und +20° C, oder in wäßriger Natriumbicarbonatlösung bei 0° C bis +10° C, mit Aminosäuren, Diaminosäuren oder Dipeptiden zu entsprechenden geschützten N-[(2,3-Di(benzyloxy)benzoyl]-aminosäuren umgesetzt werden und diese dann nach üblichen Verfahren zur Entfernung der Schutzgruppen, beispielsweise durch katalytische Hydrierung in Ethanol, in die freien catecholsubstituierten Aminosäure- bzw. Peptidderivate (Formel I mit R² = R¹¹, R¹², R¹³ oder R¹⁴ mit Z = CH₂) umgewandelt werden,
   oder indem
e. Dihydroxy- bzw. Diacyloxybenzoylchlorid (Formel I mit R¹ = OH, OAcyl und R² = COCI) mit Oxazolidincarboxylat, erhalten nach bekannten Verfahren aus Serin und Aldehyden, beispielsweise Formaldehyd, in Alkalilauge und anschließendem Ansäuern, in einem geeigneten Lösungsmittel, beispielsweise in Ethanol oder Ethanol/Wasser-Gemisch bei einer Temperatur zwischen -10° C bis +10° C zu substituierten Oxazolidincarbonsäurederivaten (Formel I mit R² = R¹⁴ und Z = O) umgesetzt werden,
   oder indem
f. catecholsubstituierte Benzaldehyde (Formel I mit R² = CHO) in einem geeigneten Lösungsmittel mit O-Carboxymethylhydroxylamin bzw. dessen Salzen zu entsprechenden Formyl-O-carboxymethyloximen (Formel I mit R² = CH = NOCH₂COOH) umgesetzt werden.

Die erfindungsgemäßen Verbindungen der Formel I mit Y in R² = ein Wirkstoffrest, der eine freie NH- oder OH-Gruppe aufweist, werden z. B. hergestellt, indem eine Verbindung der Formel I mit Y in R² = OH, z. B. nach der Gemischtanhyclridmethode zunächst mit Chlorameisensäureester und einem tertiären Amin, z. B. Triethylamin, und dann mit dem entsprechenden Wirkstoff, der eine freie NH- oder OH-Gruppe sowie gegebenenfalls eine gebräuchliche Linkergruppe, wie z. B. Reste einer Diaminocarbonsäure, einer Hydroxycarbonsäure bzw. eines Diamins oder Diols, enthält, zusammen mit einem geeigneten tertiären Amin, z. B. Triethylamin in einem geeigneten Lösungsmittel, z. B. Tetrahydrofuran, umgesetzt wird.

Die Verbindungen der Formel I mit einer Carboxylgruppe können als freie Säuren, in Form ihrer Salze oder als leicht spaltbare, insbesondere unter physiologischen Bedingungen spaltbare, Ester vorliegen. Die Reinigung der Verbindungen erfolgt nach üblichen, aus dem Stand der Technik bekannten Verfahren, beispielsweise durch Umkristallisation oder mittels chromatographischer Methoden.

Die erfindungsgemäßen Verbindungen zeigen bei verschiedenen gramnegativen Bakterienstämmen Siderophorwirksamkeit.

Die Prüfung auf Siderophorwirksamkeit erfolgte mit verschiedenen bakteriellen Indikatormutanten, die aufgrund fehlender Siderophore nur geringes Wachstum zeigen und nach Zugabe der Testsubstanzen als Ersatzsiderophore zu einer Wachstumssteigerung befähigt werden. Bei den Indikatormutanten ist die Biosynthese der jeweiligen Siderophore, z. B. Pyoverdin, Pyochelin, Enterobactin, Aerobactin, Yersiniabactin oder die Aromatenbiosynthese geblockt oder es fehlen Rezeptoren für Enterobactin, Pyochelin bzw. Pyoverdin sowie andere wichtige Komponenten für den bakteriellen Eisentransport (z. B. Membranproteine Cir, Fiu, FepA, TonB). Diese Mutanten können deshalb unter Eisenmangelbedingungen nicht oder nur sehr gering wachsen. Im einzelnen wurden folgende Indikatormutanten verwendet: *Pseudomonas aeruginosa* PAO 6609, K 407, 690, *E*. *coli* AB 2847, *Salmonella typhimurium* enb-7, TA 2700; *Klebsiella pneumoniae* KN 4401; *Yersinia enterocolitica* WAH; *Proteus mirabilis* 12 (wild); *Proteus vulgaris* 718 (wild) und *Morganella morganii* SBK3 (wild). Die mit "wild" bezeichneten Wildstämme verfügen nur über mangelhafte Eisenaufnahmesysteme, weshalb Siderophorzugabe ein gesteigertes Wachstum bewirkt. Als Kontrollen wurden bei den *Pseudomonas*-Stämmen Ferrioxamin E, bei den *Salmonella*-Stämmen Ferrioxamin G und Enterobacbin, bei den *E. coli-*, *Klebsiella-* und Y. *enterocol*.-Stämmen Ferrichrom und bei den *Proteus-Stämmen* sowie bei *Morganella morganii* 3,4-Dihydroxybenzyliden-2,4,6-trimethylanilin (zur Literatur R. Reissbrodt et al. siehe oben) eingesetzt.

Im Falle der *E. coli-Mutanten* IR 112 und H 1728, bei denen die für einen aktiven Eisentransport wichtigen Membranproteine TonB bzw. Cir und Fiu fehlen, waren alle geprüften Substanzen wirkungslos. Dies ist ein Hinweis auf eine echte Siderophorwirksamkeit der Substanzen.

Die Wachstumszonen der Indikatormutanten (Durchmesser in mm) unter dem Einfluß der Testsubstanzen sind in den Tabellen 1 bis 3 angegegben. Die Angaben + bzw. (+) beziehen sich auf unspezifische Wachstumsförderung.

**Tabelle 1:**

| **Wachstumszonen (in mm) von Siderophor-Indikatorstämmen mit neuen synthetischen Catecholderivaten** | | | | | | |
|---|---|---|---|---|---|---|
| Substanz.-Nr. | Pseudomonas aeruginosa | | | E.coli | Salmonella typhimurium | |
| | PAO 6609 | K407 | 690 | AB 2847 | enb-7 | TA 2700 |
| a. | | | | | | |
| **1** | 20 | 16 | 23 | 0 | 35 | 0 |
| **2** | 17 | 18 | 17 | 0 | 30 | 0 |
| **3** | 0 | 0 | 0 | 22 | 28 | 0 |
| **4** | 17 | 18 | 18 | 18 | 34 | 0 |
| **5** | 0 | 0 | 0 | 15 | 0 | 0 |
| **6** | 14 | 17 | 19 | 17 | 34 | 0 |
| **7** | 0 | 0 | 0 | 0 | 0 | 0 |
| **8** | 13 | 15 | 0 | 13 | 0 | 0 |
| b. | | | | | | |
| **9** | | | 10 | 0 | 0 | 0 |
| **10** | 0 | 0 | 0 | | 22 | 20 |
| **11** | 14 | 0 | 12 | 0 | 10 | 0 |
| **12** | 14 | 0 | 12 | 14 | 0 | 0 |
| **13** | 10 | 11 | 10 | | 18 | 23 |
| **Kontrolle s. Text** | 35 | 35 | 40 | 23 | 38 | 20 |

**Tabelle 2:**

| **Wachstumszonen (in mm) von Siderophor-Indikatorstämmen mit neuen synthetischen Catecholderivaten** | | | | | |
|---|---|---|---|---|---|
| Substanz-Nr. | Klebsiella | Y.enterocol. | Proteus mir. | Proteus vul. | M.morg. |
| | KN 4401 | WAH | 12 | 718 | SBK 3 |
| a. | | | | | |
| **1** | 0 | 10 | 26 | 35 | 35 |
| **2** | 35 | 0 | 16 | 25 | 20 |
| **3** | 32 | 0 | 18 | 18 | 20 |
| **4** | 33 | 12 | 23 | 24 | 27 |
| **5** | 0 | 0 | 19 | 21 | 21 |
| **6** | 25 | | 23 | 25 | 25 |
| **7** | 23 | 0 | 20 | 22 | 22 |
| **8** | 20 | 12 | 20 | 20 | 20 |
| b. | | | | | |
| **9** | 27 | 10 | 9 | | |
| **10** | 18 | 26 | 15 | 16 | 16 |
| **11** | 32 | 10 | 15 | 20 | 20 |
| **12** | 30 | 25 | 17 | 20 | 15 |
| 13 | 15 | 10 | 23 | 23 | 24 |
| **14** | 27 | 10 | 10 | 12 | 11 |
| **15** | 0 | 12 | | | 11 |
| **Kontrolle s. Text** | 25 | 26 | 18 | 25 | 18 |

**Tabelle 3:**

| **Wachstumszonen (in mm) von Siderophor-Indikatorstämmen mit neuen synthetischen Catecholderivaten** | | | | | | | |
|---|---|---|---|---|---|---|---|
| Sub. Nr. | P. aeruginosa | | | E. coli | S. typhimurium | | M. morg. |
| | PAO 6609 | K407 | 690 | AB 2847 | enb-7 | TA 2700 | SBK3 |
| c. | | | | | | | |
| **16** | 20 | 20 | 20 | 24 | 25 | 10 | |
| **17** | 0 | | | 26 | 0 | | 12 |
| d. | | | | | | | |
| **18** | 20 | 17 | 18 | 30 | 48 | 50 | |
| **19** | | 10 | 0 | 11 | 29 | 17 | |
| **20** | | 10 | 0 | 18 | 31 | 12 | |
| **21** | 14 | 15 | 15 | 33 | 36 | 40 | |
| **22** | 48 | 40 | 50 | 0 | 50 | 50 | |
| **23** | | | 14 | 22 | 27 | 20 | |
| **24** | 20 | 20 | 20 | 40 | 50 | 40 | |
| **25** | 18 | | | 30 | 28 | | 38 |
| **26** | 0 | | | 14 | 32 | | 10 |
| **Kontrolle s. Text** | 35 | 35 | 40 | 23 | 38 | 20 | |

Aufgrund ihrer Eigenschaften als bakterielle Siderophore können die Verbindungen der allgemeinen Formel I als Transportvehikel bzw. Penetrationsbeschleuniger für antimikrobielle Antibiotika und andere Wirkstoffe dienen, d. h. sie können in Konjugaten mit Antibiotika bzw. anderen Wirkstoffen diese über Eisentransportwege in die Mikrobenzelle transportieren und somit deren Wirksamkeit erhöhen.

Verbindungen der allgemeinen Formel I mit Y in R² = ein Wirkstoffrest besitzen antibakterielle Wirksamkeit, zum Teil auch bei gegenüber anderen β-Lactamen resistenten Bakterien. Dafür wurden in einem Agardiffusionstest einige Verbindungen der allgemeinen Formel I und y = ein Wirkstoffrest, z.B. die Substanzen 28-37, gegen spezielle, gegenüber anderen ß-Lactamen zum Teil resistente Bakterienstämme getestet (Tabelle 4). Die folgenden Stämme wurden verwendet: *Pseudomonas aeruginosa* SG 137 (Carbenicillin-resistent), KW 799/WT (Wildtyp), KW 799/61 (Penetrationsmutante, Zellwand geschädigt, Penetration erleichtert), ATCC 27853 (Wildtyp), ATCC 9027 (Wildtyp), NCTC 10662 (ATCC 25668, Klinikisolat Carbenicillin-sensibel), NCTC 10701 (Carbenicillin-sensibel), NPS1 und Oxa6 (Plasmidcodierte ß-Lactamase) ; *E*. *coli* DCO (Wildtyp), DC2 (Penetrationsmutante, Zellwand geschädigt, Penetration erleichtert) , *Klebsiella pneumoniae* ATCC 10031 (Wildtyp) sowie SG 117; *Salmonella gallinarum* ATCC 9184; *Stenotrophomonas maltophilia* GN 12873 (Ampicillin-, Azlocillin- und Carbapenemresistent) und IMET 10402.

Überraschenderweise wurde gefunden, daß die getesteten Substanzen nicht nur bei den Ampicillin-resistenten und/oder β-Lactamaseinhibitor-resistenten Wildtypstämmen eine hervorragende Wirksamkeit aufwiesen, sondern daß sie auch bei zwei *Pseudomonas-*Stämmen mit plasmidcodierter β-Lactamase (NPS1, Oxa6) und multiresistenten *Stenotrophomonas-Stämmen* wirksam waren, während z.B. Azlocillin und zum Teil auch Meropenem oder Imipenem unwirksam waren.

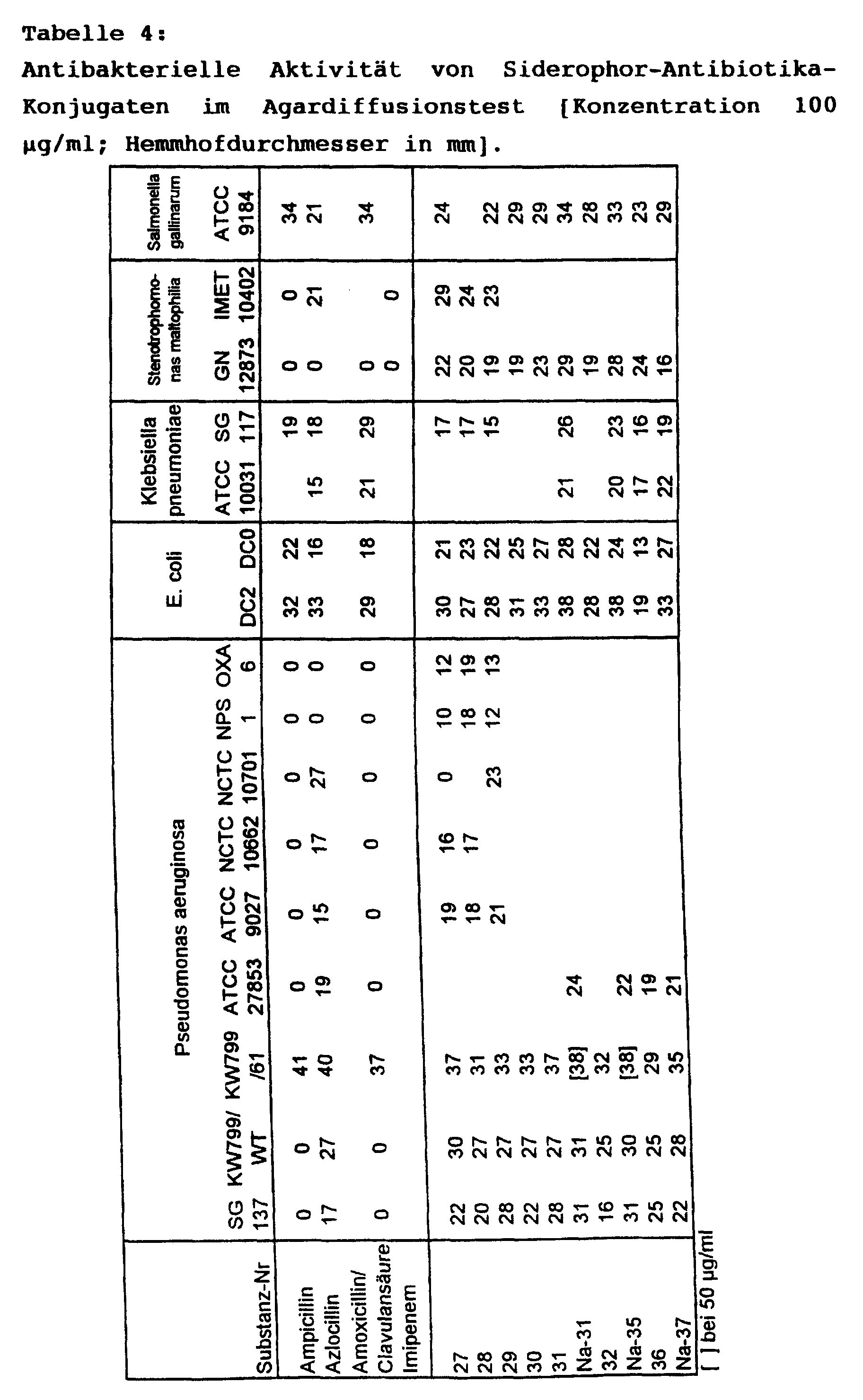

Auch in einem Mikrodilutionstest wurde die überraschend gute Wirksamkeit belegt. Die minimalen Hemmkonzentrationen (MHK) wurden bei den folgenden Bakterienstämmen bestimmt: *Pseudomonas aeruginosa* NCTC 10701, NCTC 10662, SG 137, ATCC 27853, KW 799/WT und KW 799/61; E.coli DCO, DC2 und ATCC 25922, *Serratia marcescens* SG621; *Salmonella gallinarium* ATCC 9184; *Klebsiella pneumoniae* ATCC 10031 und SG 117.

Die Ergebnisse sind in Tabelle 5 wiedergegeben. Danach sind alle Siderophor-Ainpicillin-Konjugate im Vergleich zu Ampicillin und Azlocillin als Standard gut wirksam, besonders bei *Pseudomonas aeruginosa* SG 137, einem gegenüber Carbenicillin resistenten Keim, sowie Wildtypstämmen von *Pseudomonas,* aber z.T. auch gegen E. *coli* und *Serratia.*

Mit den Testkeimen KW 799/WT und /61 von *Pseudomonas* und DC0 und DC2 von *E*. *coli* wurde der Anteil der verbesserten Penetrationsfähigkeit an der Wirksamkeit der Substanzen untersucht. Bei KW 799/61 und DC2 handelt es sich um Mutanten mit durchlässiger äußerer Membran im Vergleich zur den Wildtypen KW 799/WT bzw. DC0. Bei den Vergleichssubstanzen Azlocillin und Ampicillin ist an Hand der starken Unterschiede in der Aktivität gegen Wildtyp und Mutante die mehr oder weniger schlechte Penetrationsfähigkeit festzustellen, im Gegensatz zu den Konjugaten mit guter Penetrationsfähigkeit.

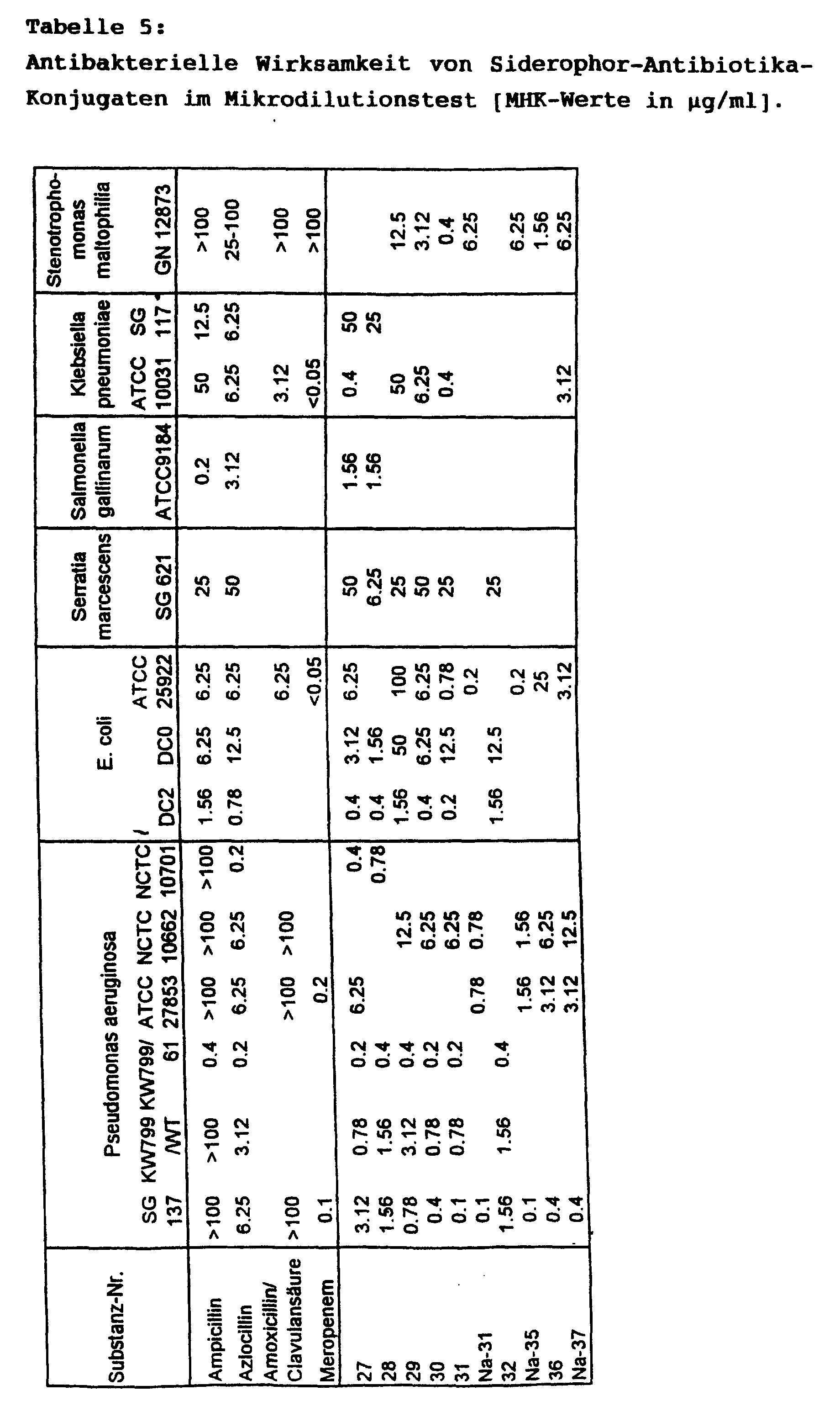

Bereits die Ergebnisse mit den Penetrationsmutanten von *Pseudomonas,* KW 799/61, und *E. coli* DC2 und deren Wildtypen belegen, daß die meisten der neuen Substanzen über eine wesentlich bessere Penetrationsfähigkeit verfügen als Ampicillin und Azlocillin. Es wurde mit Hilfe weiterer Experimente mit speziellen *E. coli*-Mutanten, denen die Porine ompC und ompF, über die die β-Lactame normalerweise in die Bakterienzelle gelangen, oder das für einen aktiven Eisentransport essentielle Membranprotein tonB fehlen, gezeigt, daß die beschriebenen Siderophor-Antibiotika-Konjugate zwei Penetrationswege nutzen können (über die Porine ompC und ompF und über den Eisentransportweg tonB), während die antibiotische Aktivität von Ampicillin und Azlocillin nur vom Vorhandensein der Porine abhängig ist. Die Wirksamkeit gegen β-Lactamasebildner und multiresistente Keime ist daher auf einen neuartigen Mechanismus zur Überwindung der Penetrationsresistenz zurückzuführen, durch den das Verhältnis von Wirkstoff zu Enzym in der Bakterienzelle so beeinflußt wird, daß nicht alle Antibiotikummoleküle inaktiviert werden, bevor sie ihr Target erreichen.

**Tabelle 6:**

| Abhängigkeit der antibakteriellen Aktivität der neuen Substanzen von Porinen und TonB bei Mutanten von E. *coli* (Hemmhofdurchmesser im Agardiffusionstest in mm) | | | | | |
|---|---|---|---|---|---|
| **Substanz- Nr** | **Mutante KB5 ompC-** | **KB4 ompF-** | **PLB3268** | **BR185** | **AB2847** |
| | | | **ompF++** | **tonB-** | **tonB+** |
| 27 | 7.5 | 8.5 | 14 | 1 | 7 |
| 28 | 11 | 11.5 | 13 | 0 | 8.5 |
| 29 | 7 | 9.5 | 14 | 0 | 6 |
| Ampicillin | 9.5 | 10 | 17.5 | 12.5 | 12.5 |
| Azlocillin | 4 | 6.5 | 17.5 | 8 | 8 |
| **PLB268: ompF- wird überexprimiert** | | | | | |

In Tabelle 7 sind weiterhin die Ergebnisse des CAS-Testes verzeichnet. Der CAS-Test (Chromazurol-S-Test) nach Schwyn und Neilands (Anal. Biochem. 160, 47 (1987) beruht auf einer Farbreaktion bedingt durch die Ablösung des Fe aus dem Chromazurol-S-Komplex und die Bindung durch die Catecholverbindung, womit die Siderophoreigenschaft der Verbindung nachgewiesen wird. Die CAS-Tests waren bei den neuen Substanzen positiv, währen sie mit Ampicillin und Azlocillin völlig negativ verliefen. Auch dies bestätigt den überraschenden Befund, daß die neuen Antibiotika zusätzlich zum Porinweg verstärkt über den Eisentransportweg in die Bakterienzelle gelangen.

**Tabelle 7:**

| **Fe-Komplexierung durch neuartige Antibiotika (CAS-Test)** | |
|---|---|
| **Substanz-Nr.** | **CAS-Test** |
| 27 | ++ |
| 28 | ++ |
| 29 | +++ |
| Ampicillin | - |
| Azlocillin | - |

Verbindungen der allgemeinen Formel I, sowie beim Vorliegen saurer Gruppen auch deren Salze und unter physiologischen Bedingungen spaltbare Ester, eignen sich aufgrund ihrer Eigenschaften als Siderophore bzw. Eisenchelatoren zur Anwendung als Arzneimittel bei Krankheiten, die auf einer Störung des physiologischen Eisenstoffwechsels beruhen. Verbindungen der allgemeinen Formel I mit Y in R² = ein Wirkstoffrest, z. B. der Rest eines Antibiotikums mit einer NH- oder OH-Gruppe, sowie beim Vorliegen saurer Gruppen auch deren Salze und unter physiologischen Bedingungen spaltbare Ester, eignen sich aufgrund ihrer antibakteriellen Wirksamkeit zur Anwendung als Arzneimittel gegen bakterielle Infektionen bei Menschen und Nutztieren.

Bei den genannten Erkrankungen können die Verbindungen der Formel I entweder allein oder in Form von pharmazeutischen Präparaten mit physiologisch verträglichen, aus dem Stand der Technik bekannten Hilfs- oder Trägerstoffen angewandt werden, wobei prinzipiell alle üblichen pharmakologischen Anwendungsformen möglich sind.

### Beispiele

### a. Aromatische Azomethincarbonsäuren

### Allgemeine Vorschrift für die Beispiele 1, 2 und 7:

2 mmol des jeweiligen Benzaldehyds und 2 mmol der entsprechenden Aminobenzoesäure wurden in 100 ml getrocknetem Toluol 4 bis 5 Stunden am Wasserabscheider unter Rückfluß erhitzt. Die nach Abkühlung auf Raumtemperatur bzw. Einengen des Lösungsmittels ausgefallenen Kristalle wurden abgesaugt und umkristallisiert.

### Allgemeine Vorschrift für die Beispiele 3, 4, 5, 6 und 8:

2 mmol des jeweiligen Benzaldehyds und 2 mmol der entsprechenden Aminobenzoesäure (1 mmol bei Diaminoverbindungen) wurden in 80 ml trockenem Ethanol 3 bis 4 Stunden unter Rückfluß erhitzt. Dazu wurde ein mit Molekularsieb gefüllter Soxhlet-Extraktionsaufsatz zur Bindung des bei der Reaktion entstehenden Wassers benutzt. Die nach Abkühlung auf Raumtemperatur bzw. Einengen des Lösungsmittels ausgefallenen Kristalle wurden abgesaugt und umkristallisiert.

### Beispiel 1

### 3-[3,4-Di(methoxycarbonyloxy)-benzylidenamino]-benzoesäure (1)

Formel I mit R¹ = OCOOCH₃, R² = R⁷ in 4-Position, mit R³ = H, COY in 3-Position, X = CH, Y = OH Substanz (1) wurde durch Umsetzung von 3, 4-Di (methoxycarbonyloxy) -benzaldehyd und 3-Aminobenzoesäure mit einer Ausbeute von 53 % der Theorie als schwachgelber Feststoff und einem Pf. 198 bis 199° C (Toluol) erhalten.

### Beispiel 2

### 3-[3,4-Di (methoxycarbonyloxy)benzylidenamino]-4-hydroxybenzoesäure (2)

Formel I mit R¹ = OCOOCH₃, R² = R⁷ in 4-Position, mit R³ = 2-OH, COY in 5-Position, X = CH, W = OH

Substanz (2) wurde durch Umsetzung von 3,4-Di(methoxycarbonyloxy)-benzaldehyd und 3-Amino-4-hydroxy-benzoesäure mit einer Ausbeute von 30 % der Theorie als schwachgelber Feststoff und einem Fp. 221 bis 223° C (Toluol) erhalten.

### Beispiel 3

### 3-[2, 3-Dihydroxy)benzylidenamino]-4-hydroxybenzoesäure (3)

Formel I mit R¹ = OH, R² = R⁷ in 3-Position, mit R³ = 2-OH, COY in 5-Position, X = CH, Y = OH

Substanz (3) wurde durch Umsetzung von 2,3-Dihydroxybenzaldehyd und 3-Amino-4-hydroxybenzoesäure mit einer Ausbeute von 59 % der Theorie als rote Kristalle und einem Fp. 273 bis 274° C (Ethanol) erhalten.

### Beispiel 4

### 3,5-Bis-[3,4-di (methoxycarbonyloxy)-benzylidenamino]-benzoesäure (4)

Formel I mit R¹ = OCOOCH3, R² = R⁷ in 4-Position, mit 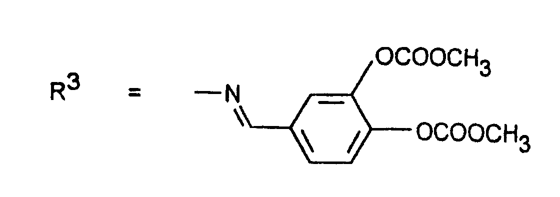 in 3-Position,
COY in 5-Position, X = CH, Y = OH

Substanz (4) wurde durch Umsetzung von 3,4-Di(methoxycarbonyloxy)benzaldehyd und 3,5-Diamino-benzoesäure mit einer Ausbeute von 49 % der Theorie als schwachgelber Feststoff mit einem Fp. 145 bis 148° C (Toluol) erhalten.

### Beispiel 5

### 4-[2,3-Dihydroxy)benzylidenamino]-3-hydroxybenzoesäure (5)

Formel I mit R¹ = OH, R² = R⁷ in 3-Position, mit R³ = 2-OH, COY in 4-Position, X = CH, Y = OH

Substanz (5) wurden durch Umsetzung von 2,3-Dihydroxybenzaldehyd und 4-Amino-3-hydroxybenzoesäure mit einer Ausbeute von 85 % der Theorie als rote Kristalle und einem Fp. 278 bis 280° C (Ethanol) erhalten.

### Beispiel 6

### 4- (3, 4-Diacetoxybenzylidenamino) -benzoesäure (6)

Formel I mit R¹ = OCOCH₃, R² = R⁷ in 4-Position, mit R³ = H, COY in 4-Position, X = CH, Y = OH

Substanz (6) wurde durch Umsetzung von 3,4-Diacetoxybenzaldehyd und 4-Aminobenzoesäure mit einer Ausbeute von 77 % der Theorie als gelber Feststoff mit einem Fp. 180 bis 182° C (Toluol) erhalten.

### Beispiel 7

### 3-[(3,4-Diacetoxyphenylimino)-methyl]-4,5-dihydroxybenzoesäure (7)

Formel I mit R¹ = OCOCH₃, R² = R⁸ in 3-Position, mit R³ = 2, 3-OH, COY in 5-Position, Y = OH

Substanz (7) wurde durch Umsetzung von 3-Formyl-4, 5-di-hydroxybenzoesäure und 3,4-Di(acetoxy) anilin mit einer Ausbeute von 79 % der Theorie als rote Kristalle mit einem Fp. 240 bis 243° C erhalten.

¹H-NMR (Dioxan-d₈, δ in ppm): 8,79 (s, 1 H, CH = N), 7,76 (s, 1 H, ArH), 7,56 (s, 1 H, ArH), 7,28 (m, 3 H, ArH), 2,26 (s, 3 H, CH₃CO), 2,24 (s, 3 H, CH₃CO).

### Beispiel 8

### 3-(3,4-Dihydroxyphenylazo)benzoesäure (8)

Formel I mit R¹ = OH, R² = R⁷ in 4-Position, mit X = N, R₃ = H, COY in 3-Position, Y = OH

3-Aminobenzoesäure (612 mg, 4,5 mmol) wurde in 15 ml Ethanol und 2,5 ml konzentrierter Salzsäure mit 312 mg Natriumnitrit bei 0° C diazotiert. Zu der Lösung des Diazoniumsalzes gab man unter Rühren bei 0° C eine Lösung von 0,97 g (4,5 mmol) Brenzcatechinmonobenzoat in 20 ml Ethanol zu. Anschließend wurden noch 5 ml einer 25 %-igen Na₂CO₃-Lösung zugegeben, wobei der pH-Wert bei 9 liegen sollte. Es wurde noch 2 Stunden bei 0° C gerührt, mit Salzsäure auf pH 2 gestellt und die Reaktionsmischung mit Dichlormethan extrahiert. Die organische Phase wurde mit Wasser gewaschen und über Natriumsulfat getrocknet. Das nach Entfernung des Lösungsmittels im Vakuum erhaltene Rohprodukt wurde aus Ethanol/Wasser umkristallisiert. Ausbeute: 327 mg (28 % der Theorie). Rotbraune Kristalle, Fp. : 213 bis 215° C (Ethanol).
¹H-NMR (DMSO-d₆) δ in ppm; J in Hz) : 8,28 (m, 1 H, ArH), 8,04 (dd, J = 1,8, 7,8, 2 H, ArH) , 7,69 (t, J = 7,8, 1 H, ArH), 7,42 (dd, J = 2,3, 8,3, 1 H, ArH), 7,36 (d, J = 2,3, 1 H, ArH), 6,94 (d, J = 8,3, 1 H, ArH).

### Benzhydrazone

### Beispiel 9

### [(3,4-Dihydroxybenzoyl)-hydrazono]-phenylessigsäure (9)

Formel I mit R¹ = OH, R² = R⁹ in 4-Position, mit R⁴ = COOH, R⁵ = H, R¹⁵ = H

1,2 g Phenylglyoxylsäure (0,01 Mol) in 5 ml Ethanol und 1,7 g 3, 4-Dihydroxybenzhydrazid (0, 01 Mol) in 5 ml 2 N Salzsäure wurden 1 Stunde bei Raumtemperatur gerührt. Farblose Kristalle (Ethanol), Ausbeute 1,18 g (40 % der Theorie), Fp. 194 bis 195° C (Zersetzung).
¹H-NMR (DMSO-d₆, δ in ppm) : 12,8 (s, 1 H, NHCO), 6,86 bis 7,69 (m, 8 H, ArH)

### Beispiel 10

### 4-[(2,3-Dihydroyxybenzoyl)-hydrazonomethyl] -benzoesäure (10)

Formel I mit R¹ = OH, R² = R⁹ in 3-Position, mit R⁴ = H, R¹⁵ = H, R⁵ = 4-COOH

0,6 g (5 mmol) 4-Formylbenzoesäure und 0,85 g (5 mmol) 2,3-Dihydroxybenzhydrazid wurden in 10 ml Ethanol 1 Stunde unter Rückfluß gekocht. Farblose Kristalle (Ethanol/Wasser), Ausbeute 909 mg (61 der Theorie), Fp. 301 bis 302° C.

### Beispiel 11

### 4-[(3,4-Dihydroxybenzoyl) -hydrazonomethyl]-benzoesäure (11)

Formel I mit R¹ = OH, R² = R⁹ in 4-Position, mit R⁴ = H, R¹⁵ = H, R⁵ = 4-COOH

0,6 g (5mmol) 4-Formylbenzoesäure und 0,85 g (5 mmol) 3,4-Dihydroxybenzhydrazid wurden in 10 ml Ethanol 1 Stunde unter Rückfluß gekocht. Farblose Kristalle, Reinigung durch Lösen in Dimethylformamid, Filtrieren und Ausfällen mit Wasser, Ausbeute 889 mg (61 % der Theorie), Fp. 314 bis 315° C

### Beispiel 12

### {[3,4-Di-(methoxycarbonyloxy)-benzoyl]-hydrazon}phenylessigsäure (12)

Formel I mit R¹ = OCOOCH₃, R² = R⁹ in 4-Position, mit R⁴ = COOH, R⁵ = H, R¹⁵ = H

300 mg (1 mmol) [(3,4-Dihydroxybenzoyl)-hydrazono]phenylessigsäure = Produkt aus Beispiel 9 (1 mmol) wurden in 2 ml 2 N Natronlauge und 3 ml Wasser bei 0° C mit 2 ml Chlorameisensäuremethylester 10 Minuten gerührt. Dann wurde mit 2 N Natronlauge alkalisch gemacht, 1 ml Chlorameisensäuremethylester zugegeben und nochmals 30 Minuten gerührt. Anschließend wurde mit 2 N Salzsäure auf pH 2 gebracht. Farblose Kristalle (aus Methanol/Wasser Ausbeute 221 mg (53 % der Theorie), Fp. 171 bis 172° C (Zersetzung).
¹H-NMR (DMSO-d₆, δ in ppm): 7,4 - 7,9 (m, 8 H, ArH), 3,87, 3,85 (s, 2 x 3 H OCOCH₃)

### Beispiel 13

### 3-Formyl-4, 5-dihydroxybenzoesäure- (2, 3-dihydroxybenzhydrazon) (13)

Formel I mit R¹ = OH, R² = R⁹ in 3-Position, mit R⁵ = 5-COOH, R⁴ = H, R¹⁵ = 3,4-OH

Eine Mischung von 364 mg 3-Formyl-4,5-dihydroxybenzoesäure wurden in heißem Wasser gelöst und 340 mg 2, 3-Dihydroxybenzhydrazid in 5 ml 2 N Salzsäure gelöst und 10 Minuten unter Rühren gekocht. Farblose Kristalle (Eisessig), Ausbeute 431 mg (65 der Theorie), Fp = 280 - 281° C.
¹H NMR (DMSO-d6, δ in ppm) : 8, 72 (s, 1 H, CH = N), 6,8 - 7,6 (m, 5 H, ArH), 7,74 - 7,75 (2 x 1H, d, J = 1,6, 2- bzw. 6 CH von Benzoesäure), 7,35 - 7,39 und 6,98 - 7,02 (2 x 1 H, d, J = 8,4 und 6-CH von Benzhydrazon), 6,74 - 6,82 (1H, t, J = 8,5, CH von Benzhydrazon)

### Beispiel 14

### 4-{[3,4-Di-(methoxycarbonyloxy)-benzoyl]-hydrazonomethyl)-benzoesäure (14)

Formel I mit R¹ = OCOOCH₃, R² = R⁹ in 4-Position, mit R⁵ = 4-COOH, R⁴ = H, R¹⁵ = H

300 mg (1 mmol) 4-[(3,4-Dihydroxybenzoyl)-hydrazonomethyl]-benzoesäure = Produkt aus Beispiel 11 wurden in 1 ml 2 N Natronlauge und 3 ml Wasser gelöst, die Lösung wurde auf 0° C gekühlt und unter Rühren mit 2 ml Chlorameisensäuremethylester versetzt. Die Mischung wurde weitere 30 Minuten bei 0° C gerührt und dann mit Salzsäure auf pH 3 gebracht. Farblose Kristalle (Ethanol/Wasser), Ausbeute 205 mg (49 % der Theorie), Fp. 184 bis 187° C (Zersetzung).
¹H-NMR (DMSO-d6, δ in ppm): 8,50 (s, 1 H, CH = N), 7,62 - 8,04 (m, 7 H, ArH), 3, 88 (s, 6 H, 2 x OCOOCH₃)

### Beispiel 15

### 6-[(3,4-Dihydroxybenzoyl)-hydrazonomethyl]-2,3-dihydroxybenzoesäure (15)

Formel I mit R¹ = OH, R² = R⁹ in 4-Position, mit R⁵ = 2-COOH, R⁴ = H, R¹⁵ = 3,4-OH

Eine Mischung von 182 mg (1 mmol) 6-Formyl-2,3-dihydroxybenzoesäure in heißem Wasser gelöst und 168 mg (1 mmol) 3,4-Dihydroxybenzhydrazid, in 5 ml 2 N Salzsäure gelöst, wurde 10 Minuten unter Rühren gekocht. Blaßgelbe Kristalle (Wasser). Ausbeute 215 mg (65 % der Theorie), Fp. 252° C
¹H-NMR (DMSO-d6, δ in ppm): 8,53 (s, 1 H, CH = N) , 6,8 - 7,3 (m, 5 H, ArH)

### c. Aminobenzoesäurederivate

### Beispiel 16

### 2-(2,3-Diacetoxy-benzoylamino)-benzoesäure (16)

Formel I mit R¹ = OCOCH₃, R² = R¹⁰ in 3-Position, mit COY in 2-Position, Y = OH, R¹⁸ - R²⁰ = H

### Verfahren 1:

1,50 g (0,011 Mol) Anthranilsäure wurden in 100 ml 0,5 M NaHCO₃-Lösung aufgeschlämmt und bei 0 bis 10° C im Ultraschallbad unter Rühren mit 2,56 g (0,01 Mol) 2,3-Diacetoxybenzoylchlorid in 8 ml Tetrahydrofuran versetzt. Die nach 45 Minuten gebildete trübe Lösung wurde filtriert und mit konzentrierter Salzsäure vorsichtig angesäuert. Die erhaltenen farblosen Kristalle wurden mit wenig Ethylacetat gewaschen und im Vakuum getrocknet. Fp. 203 bis 204° C, Ausbeute 2,7 g (75 % der Theorie)
¹H-NMR (DMSO-d6, δ in ppm) : 11, 8 (s, 1 H, NHCO), 7,2 - 8,5 (m, 7 H, ArH), 2,15 und 2,25 (s, 2 x 3 H, OCOCH₃)

### Verfahren 2:

Zu 1,4 g (0,01 Mol) Anthranilsäure in 100 ml Natriumbicarbonatlösung wurden bei 0° C 2,57 g 2,3-Diacetoxybenzoylchlorid in 50 ml Tetrahydrofuran gegeben. Die Mischung wurde 1 Stunde bei 0 bis 10° C und anschließend 30 Minunten bei Raumtemperatur im Ultraschallbad zur Reaktion gebracht. Anschließend wurde teilweise eingeengt und vorsichtig mit 2 N Salzsäure angesäuert. Blaßgelbe Kristalle (Ethylacetat), Ausbeute 2,3 g (64 % der Theorie), Fp. 198° bis 200° C

### Beispiel 16a

### 4-[(2,3-Di-methoxycarbonyloxy-benzoyl)-methylamino]-benzoesäure

Formel I mit R¹ = OCOOCH₃, R² = R¹⁰ in 3-Position, R¹⁹ = CH₃, R¹⁸ = R²⁰ = H, COY in 4-Position, Y = OH

Unter Anwendung des Verfahrens 1 erhielt man analog aus 4-Methylamino-benzoesäure und 2,3-Di-(methoxycarbonyloxy)-benzoylchlorid die Titelverbindung in einer Ausbeute von 65 % der Theorie

### Beispiel 16b

### 3,5-Bis-(2,3-diacetoxy-benzoylamino)-benzoesäure

Formel I mit R¹ = OCOCH₃, R² = R¹⁰ in 3-Position, R¹⁸ = R¹⁹ = H, R²⁰ = (2,3-Diacetoxy)-benzoylamino in 5-Position, COY in 3-Position, Y = OH

Unter Anwendung des Verfahrens 1 erhielt man analog aus 3,5-Diaminobenzoesäure und 2, 3-Diacetoxybenzoylchlorid die Titelverbindung in einer Ausbeute von 55 % der Theorie

### Beispiel 17

### 4-(2,3-Diacetoxy-benzoylamino)-benzoesäure (17)

Formel I mit R¹ = OCOCH₃, R² = R¹⁰ in 3-Position, mit COY in 4-Position, Y = OH, R¹⁸ - R²⁰ = H

Zu 137 mg (1 mmol) 4-Aminobenzoesäure und 0,14 ml Triethylamin in 10 ml Tetrahydrofuran wurden bei - 20° C 257 mg 2,3-Diacetoxybenzoylchlorid in 5 ml Tetrahydrofuran gegeben. Die Mischung wurde 30 Minuten bei 20° C und anschließend 30 Minuten bei Raumtemperatur gerührt. Anschließend wurde teilweise eingeengt und vorsichtig mit 2 N Salzsäure angesäuert. Blaßgelbe Kristalle (Ethylacetat), Ausbeute 215 mg (60 % der Theorie), Fp. 134 - 135° C.
¹H-NMR (DMSO-d6, δ in ppm) : 7,3 - 7,9 (m, 7 H, ArH), 2,1 und 2,3 (s, 2 x 3 H, OCOCH₃).

### d. Aminosäurederivate:

Allgemeine Vorschrift für die Beispiele 19, 20, 22, 23 und 25:

### 1. Stufe (Acylierung):

2,5 mmol der jeweiligen Aminosäure oder des Dipeptids (in freier Form oder als Hydrochlorid bzw. -acetat vorliegend) wurden in 10 ml wäßriger NaOH-Lösung (2,5 mmol NaOH für Alanin, 7,6 mmol für Diaminosäuren, 11,5 mmol für das Dipeptid) gelöst. Bei 0° C tropfte man unter Rühren eine Lösung von 2,3-Di(benzyloxy)-benzoylchlorid (2, 5 mmol für Alanin, 5,1 mmol für Diaminosäuren, 9 mmol für das Dipeptid) in 10 ml THF langsam zu und erwärmte danach auf 20 bis 25° C. Bei dieser Temperatur wurde 4 Stunden gerührt und das Reaktionsgemisch anschließend mit 2 M Salzsäure auf pH 2 eingestellt. Man extrahierte das Reaktionsgemisch mit Ethylacetat, wusch die organische Phase mit gesättigter wäßriger Natriumchloridlösung, trocknete über Natriumsulfat und entfernte das Lösungsmittel im Vakuum. Das Rohprodukt ließ sich durch Umkristallisation (Ethanol/Wasser im Falle des Alaninderivates) oder Säulenchromatographie (Kieselgel 60, Merck, Eluent: Chloroform : Ethylacetat : Eisessig = 30 : 10 : 1 oder Ethylacetat : Toluol : Eisessig = 10 : 10 : 2) reinigen.

### 2. Stufe (Debenzylierung) :

500 mg des in der 1. Stufe angefallenen Produktes wurden in einem Gemisch von 9 ml Ethanol und 1 ml Eisessig gelöst, mit 50 mg Palladium auf Aktivkohle (10 % Pd) versetzt und bei 20° C unter Normaldruck in einer Wasserstoffatmosphäre bis zur Aufnahme der erforderlichen stöchiometrischen Menge Wasserstoff (normalerweise 4 bis 6 Stunden) gerührt. Dann wurde das Reaktionsgemisch über Celite filtriert, das Lösungsmittel abdestilliert und der Rückstand im Vakuum getrocknet.

Allgemeine Vorschrift für die Beispiele 21, 24 und 26:

### 1. Stufe (Acylierung):

2 mmol Aminosäurebenzylester-hydrochlorid oder -tosylat wurden in 10 ml Dichlormethan gelöst und die Lösung mit 4 mmol Triethylamin (8 mmol im Falle der Diaminosäurederivate) versetzt. Bei -30° C tropfte man eine Lösung von 2 mmol 2,3-Diacetoxybenzoylchlorid (4 mmol für Diaminosäurederivate) in 10 ml Dichlormethan langsam zu. Anschließend wurde noch jeweils 1 Stunde bei -30° C und bei 20 bis 25° C gerührt. Die Reaktionslösung wurde nacheinander mit 1 M Salzsäure gesättigter wäßriger Natriumhydrogencarbonatlösung und gesättigter wäßriger Natriumchloridlösung gewaschen. Die organische Phase wurde über Natriumsulfat getrocknet, das Lösungsmittel im Vakuum entfernt und der Rückstand durch Umkristallisation aus Ethanol oder Toluol (im Falle des Serinderivats) bzw. Säulenchromatographie (Lysinderivat; Kieselgel 60, Merck, Laufmittel: Ethylacetat : Toluol = 2 : 1) gereinigt.

### 2. Stufe (Benzylesterspaltung):

1 g des in der 1. Stufe angefallenen Produktes wurde in einem Gemisch aus 20 ml Ethanol und 1 ml Eisessig gelöst, mit 100 mg Palladium auf Aktivkohle (10 % Pd) versetzt und bei 20° C unter Normaldruck in einer Wasserstoffatmosphäre 2 Stunden gerührt. Das Reaktionsprodukt wurde über Celite filtriert und das Lösungsmittel im Vakuum entfernt. Der Rückstand wurde in Ethylacetat aufgenommen, mit gesättigter wäßriger Natriumchloridlösung gewaschen und die organische Phase über Natriumsulfat getrocknet. Nach Entfernung des Lösungsmittels im Vakuum kristallisierte das Produkt aus bzw. wurde durch Umkristallisation gereinigt.

### Beispiel 18

### L-2,5-Bis-(2,3-dihydroxybenzoylamino)-pentansäure (18)

Formel I mit R¹ = OH, R² = R¹¹ in 3-Position, mit R⁶ = R¹², n = 3, R¹⁵ = 2,3-OH, Y = OH, L-Form

Substanz (18) wurde aus L-Ornithin-Monohydrochlorid mit einer Ausbeute von 66 der Theorie als weißer Feststoff erhalten.
¹H-NMR (DMSO-d₆, δ in ppm) : 7,41 (dd, 1 H, ArH), 7,27 (dd, 1 H, ArH), 6,92 (m, 2 H, ArH), 6,69 (m, 2 H, ArH), 4,46 (m, 1 H, CH-N), 3,36 (m, 2 H, CH₂-N), 1,84 (m, 2 H, CH₂), 1,65 (m, 2 H, CH₂).

### Beispiel 19

### L-2-(2,3-Dihydroxybenzoylamino)-propionsäure (19)

Formel I mit R¹ = OH, R² = R¹¹ in 3-Position, mit R⁶ = CH₃, Y = OH, L-Form

Substanz (19) wurde aus L-Alanin mit einer Ausbeute von 88 % der Theorie als weißer Feststoff erhalten.
¹H-NMR (DMSO-d₆, δ in ppm): 7,40 (dd, 1 H, ArH), 6,94 (dd, 1 H, ArH), 6,72 (dd, 1 H, ArH), 4,44 (m, 1 H, CH), 1,42 (d, 3 H, CH3)

### Beispiel 20

### L-2-(2,3-Diacetoxybenzoylamino)-propionsäure (20)

Formel I mit R¹ = OCOCH₃, R² = R¹¹ in 3-Position, mit R⁶ = CH₃, Y = OH, L-Form

Substanz (20) wurde aus L-Alaninbenzylester-hydrochlorid mit einer Ausbeute von 75 % der Theorie als farblose Kristalle mit einem Fp. von 109° bis 111° C erhalten.
¹H-NMR (DMSO-d₆, δ in ppm) : 7,50 (dd, 1 H, ArH), 7, 39 (m, 2 H, ArH), 4,33 (m, 1 H, CH), 2,29 (s, 3 H, CH₃CO), 2,23 (s, 3 H, CH₃CO), 1,34 (d, 3 H, CH₃).

### Beispiel 21

### L-2,4-Bis-(2,3-dihydroxybenzoylamino)-buttersäure (21)

Formel I mit R¹ = OH, R² = R¹¹ in 3-Position, mit R⁶ = R¹², n = 2, R¹⁵ = 2,3-OH, Y = OH, L-Form

Substanz (21) wurde aus L-2,4-Diaminochlorsauredihydrochlorid mit einer Ausbeute von 81 % der Theorie als grauweißer Feststoff erhalten.
¹H-NMR (DMSO-d₆, δ in ppm): 7,42 (dd, 1 H, ArH), 7,27 (dd, 1 H, ArH), 6,94 (m, 2 H, ArH), 6,72 (m, 2 H, ArH), 4,48 (m, 1 H, CH-N), 3,42 (m, 2 H, CH₂), 2,21 (m, 1 H, CH₂), 2,05 (m, 1 H, CH₂).

### Beispiel 22

### L-3-[2,6-Bis-(2,3-dihydroxybenzoylamino)-hexanoylamino]-2-(2, 3-dihydroxybenzoylamino)-propionsäure (22)

Formel I mit R¹ = OH, R² = R¹¹ in 3-Position, mit R⁶ = R¹³, n₁ = 1, n₂ = 4, R¹⁵ = 2,3-OH, Y = OH, L-Form

Substanz (22) wurde aus L-2-Amino-3-(2,6-diaminohexanoylamino)-propionsäure mit einer Ausbeute von 70 % der Theorie als weißer Feststoff erhalten.
¹H-NMR (DMSO-d₆, δ in ppm): 7,42 (dd, 1 H, ArH), 7,26 (m, 2 H, ArH), 6,91 (m, 3 H, ArH), 6,69 (m, 2 H, ArH), 6,67 (m, 3 H, ArH), 4,55 - 4,28 (m, 3 H, CH-N, CH₂-N), 4, 10 - 3,09 (m, 3 H, CH-N, CH₂-N), 1,72 (m, 2 H, CH₂), 1,51 - 1,28 (m, 4 H, CH₂)

### Beispiel 23

### L-2-[2,3-(Diacetoxybenzoylamino)-3-hydroxypropionsäure (23)

Formel I mit R¹ = OCOCH₃, R² = R¹¹ in 3-Position, mit R⁶ = CH₂ = OH, Y = OH, L-Form

Substanz (23) wurde aus L-Serinbenzylester-hydrochlorid mit einer Ausbeute von 53 % der Theorie als farblose Nadeln mit einem Fp. von 165 bis 168° C (Aceton/n-Hexan) erhalten.
¹H-NMR (DMSO-d₆, δ in ppm): 7,59 (dd, 1 H, ArH, 7,42 (m, 2 H, ArH), 4,42 (m, 1 H, CH), 3,76 (m, 2 H, CH₂O), 2,29 (s, 3 H, CH₃CO), 2,28 (s, 3 H, CH₃CO).

### Beispiel 23a

### L-3-Benzyloxy-2-(2,3-diacetoxy-benzoylamino)-propionsäure

Formel I mit R¹ = OCOCH3, R² = R¹¹ in 3-Position, R⁶ = CH₂OCH₂ (C₆H₅), Y = OH, L-Form

Die Substanz wurde aus O-Benzyl-L-serinbenzylester Hydrochlorid analog Substanz 23, jedoch in Stufe 2 unter Verwendung von Palladium auf Aktivkohle (10 % Pd) und Cyclohexadien, in Form weißer Kristalle erhalten.

### Beispiel 24

### D-2,5-Bis- (2,3-dihydroxybenzoylamino)-pentansäure (24)

Formel I mit R¹ = OH, R² = R¹¹ in 3-Position, mit R⁶ = R¹², n = 3, R¹⁵ = 2,3-OH, Y = OH, D-Form

Substanz (24) wurde aus D-Ornithin-Monohydrochlorid mit einer Ausbeute von 66 % der Theorie als weißer Feststoff erhalten.
¹H-NMR (DMSO-d₆, δ in ppm) : 7,41 (dd, 1 H, ArH), 7,27 (dd, 1 H, ArH), 6,93 (m, 2 H, ArH), 6, 70 (m, 2 H, ArH), 4, 46 (m, 1 H, CH-N), 3, 33 (m, 2 H, CH₂-N), 1, 84 (m, 2 H, CH₂), 1, 64 (m, 2 H, CH₂).

### Beispiel 25

### L-2, 6-Bis- (2, 3-diacetoxybenzoylamino) -hexansäure (25)

Formel I mit R¹ = OCOCH3, R² = R¹¹ in 3-Position, mit R⁶ = R¹², n = 4, R¹⁵ = 2,3-OCOCH₃, Y = OH, L-Form

Substanz (25) wurde aus L-Lysinbenzylester-ditosylat mit einer Ausbeute von 71 % der Theorie als weißer Feststoff erhalten.
¹H-NMR (CDCl₃, δ in ppm, J in Hz) : 7, 65 (dd, J = 1,9, 7,4, 1 H, ArH), 7,47 (dd, J = 2,3, 7,2, 1 H, ArH), 7,32 - 7,22 (m, J = 1,8, 7,3, 5 H, ArH, CONH, 6,59 (t, J = 5,7, 1 H, CONH), 4,75 (m, J = 5,2 7,2, 1 H, CH-N), 3, 38 (m, 2 H, CH₂-N), 2, 34 (s, 3 H, CH₃CO), 2,30 (s, 3 H, CH₃CO), 2,30 (s, 3 H, CH₃CO), 2, 29 (s, 3 H, CH₃CO), 1,99 (m, 1 H, CH₂), 1,85 (m, 1 H, CH₂), 1,60 (m, 2 H, CH₂), 1,28 (m, 2 H, CH₂).

### e. Oxazolidincarbonsäurereste

### Beispiel 26

### (S)-3-(2,3-Diacetoxybenzoyl)-oxazolidin-4-carbonsaure (26)

Formel I mit R¹ = OCOCH₃, R² = R¹⁴ in 3-Position, mit Z = O, R¹⁶ = R¹⁷ = H, Y - OH, S-Form

L-Serin (105 mg, 1 mmol) wurde in 0,5 ml 2 M Natronlauge gelöst und bei 0° C mit 0,1 ml wäßriger Formaldehyd-Lösung (36,5 %) versetzt. Man ließ das Reaktionsgemisch 24 Stunden bei 0° C stehen, fügte anschließend 84 mg (1 mmol) Natriumhydrogencarbonat und 1 ml Aceton hinzu und kühlte auf -5° C. Unter Rühren wurden portionsweise 257 mg (1 mmol) 2,3-Di(acetoxy)benzoylchlorid zugegeben. Nach 1 Stunde Rühren bei -5° bis 0° C wurde mit 10 ml Wasser verdünnt und das Reaktionsgmeisch mit Diethylether extrahiert. Die wäßrige Phase wurde mit 1 M Salzsäure auf pH 2 - 3 angesäuert und erneut mit Diethylether (3 x 20 ml) extrahiert. Die organische Phase wurde über Natriumsulfat getrocknet und das Lösungsmittel im Vakuum entfernt. Das erhaltene Produkt wurde im Vakuum getrocknet. Ausbeute: 263 mg (78 % der Theorie). Weißer Schaum.
¹H-NMR (CDCl₃, δ in ppm): 7,35 - 7,27 (m, 3 H, ArH) , 4,90 (s, 2 H, O-CH₂-N), 4,82 (m, 1 H, CH), 4,39 (m, 1 H, CH₂), 4,28 (m, 1 H, CH₂), 2,33 (s, 3 H, CH₃), 2, 31 (s, 3 H, CH₃).

### f. Antibiotikakonjugate

### Beispiel 27

### N-[L-2-(2,3-Diacetoxybenzoylamino)-propionyl]-ampicillin

(nach IUPAC: 6-{2- [2- (2,3-Diacetoxybenzoylamino)propionylamino]-2-phenyl-acetylamino}-3,3-dimethyl-7-oxo-4-thia-1-azabicyclo[3.2.0]heptan-2-carbonsäure) (27)

Formel I mit R¹ = OCOCH₃, R² = R¹¹ in 3-Position, mit R⁶ = CH₃ (L-Form), Y = N-Ampicillino

500 mg (1, 62 mmol) L-2-(2,3-Diacetoxybenzoylamino)propionsäure (Substanz 20) wurden in 15 ml Tetrahydrofuran gelöst und dazu unter Rühren bei -20° C 0,18 ml (1,62 mmol) N-Methylmorpholin und anschließend 0,21 ml (1,62 mmol) Chlorameisensäureisobutylester zugetropft. Nach 1 Stunde Rühren wurde eine auf 0° C gekühlte Lösung von 565 mg (1,62 mmol) Ampicillintrihydrat in 5 ml 80 %-igem Tetrahydrofuran zugetropft. Die Mischung wurde 1 Stunde bei -20° C und 1 Stunde bei Raumtemperatur gerührt und dann im Vakuum eingeengt. Anschließend wurden Wasser und Ethylacetat zugegeben und mit 1 M Salzsäure vorsichtig auf pH 2 angesäuert. Die Mischung wurde bis zur vollständigen Lösung geschüttelt, die Ethylacetatphase abgetrennt, mit wäßriger Kochsalzlösung neutral gewaschen und über Natriumsulfat getrocknet. Nach Einengen im Vakuum wurde mit Petrolether ausgefällt. Ausbeute an Rohprodukt: 1 g Reinheit nach HPLC (Europher 100-7): ca. 75 %. Die Reinigung erfolgte über präparative HPLC (RP₁₈, Acetonitril/Wasser = 40 : 60 + 0,50 % Trifluoressigsäure, Flußrate 10 ml/min). Die das Produkt enthaltenden Fraktionen wurden sofort mit Ethylacetat extrahiert, die organische Phase mit Wasser gewaschen, getrocknet, eingeengt und mit Petrolether gefällt. Reinheit nach HPLC: 95 %
¹H-NMR (300 MHz, CDCl₃, δ in ppm, J in Hz): 7,63 (dd, J = 1,9, 7,4, ArH), 7,33 - 7,19 (m, 7H, ArH), 5,61 (m, J = 4,1, 2 H, CH-N), 5,42 (d, J = 4,1, 1 H, CH-S), 5,00 (m, J = 7,3, 1 H, CH-Me), 4,29 (s, 1 H, CH-COO) , 2,30 (s, 3 H, CH₃CO), 2,27 (s, 3 H, CH₃CO), 1,43 (s, 3 H, CH₃), 1,40 (d, J = 7,1, 3 H, CH₃), 1,37 (s, 3 H, CH₃).

### Beispiel 28

### N-[2-(2,3-Diacetoxybenzoylamino)-benzoyl]-ampicillin

(nach IUPAC: 6-{2-[2-(2,3-Diacetoxybenzoylamino)benzoylamino]-2-phenyl-acetylamino}-3,3-dimethyl-7-oxo-4-thia-1-aza-bicyclo[3.2.0]heptan-2-carbonsäure) (28)

Formel I mit R¹ = OCOCH₃, R² = R¹⁰ in 3-Position, R¹⁸ - R²⁰ = H, mit COY in 2-Position, Y = N-Ampicillino

Zu einer Lösung von 357 mg (1 mmol) 2-(2,3-Diacetoxybenzoylamino)-benzoesäure (Substanz 16) in 5 ml absolutem Tetrahydrofuran wurden bei -20° C zunächst 0,11 ml (1 mmol) N-Methylmorpholin sowie eine katalytische Menge 1-Dimethylaminopyridin und dann unter Rühren 126 µl Chlorameisensäureisobutylester zugefügt. Die Mischung wurde 1 Stunde bei - 20° C gerührt, dann wurde die Lösung von 371 mg (1 mmol) Ampicillin-Natriumsalz in 3 ml 80 %-igen Tetrahydrofuran portionsweise zugefügt. Es wurde 1 Stunde bei -20° C und 2 Stunden bei Raumtemperatur gerührt. Dann wurde das Lösungsmittel im Vakuum abdestilliert und zu dem Rückstand 20 ml Wasser und 20 ml Ethylacetat zugegeben. Die Mischung wurde vorsichtig mit 1 M Salzsäure auf pH 3 angesäuert und durchgeschüttelt. Die organische Phase wurde abgetrennt, dreimal mit wäßriger Kochsalzlösung gewaschen und über Natriumsulfat getrocknet. Nach Einengen wurde mit Petrolether ausgefällt. Ausbeute an Rohprodukt: 420 mg.

Das Rohprodukt wurde mittels präparativer HPLC getrennt (Nucleosil 7 C 18, Macherey & Nagel, Laufmittel Acetonitril/Wasser 50/50 + 0,05 % Trifluoressigsäure)

### 2. Fraktion

### N-[2-(2,3-Diacetoxybenzoylamino)-benzoyl]-ampicillin

Ausbeute von 420 mg Rohprodukt: ca. 90 mg HPLC (Europher 100 C 18-7, Laufmittel Acetonitril/Wasser 60/40 + 0,05 % Trifluoressigsäure.
¹H-NMR (DMSO-d₆, δ in ppm): 7,3 - 8,4 (m, 12 H, ArH), 5, 9 (d, 1 H, J = 7,7 Hz, CH), 5,53 (q, 1 H, CH), 5,42 (d, 1 H, J = 4,0 Hz, CH), 4,2 (s, 1 H, CH), 2,32, 2,24 (s, 2 x 3 H, CH₃), 1,40, 1,50 (s, 2 x 3 H, CH₃).

### Beispiel 29

### N-[4-(2,3-Diacetoxybenzoylamino)-benzoyl]-ampicillin (29)

Formel I mit R¹ = OCOCH₃, R² = R¹⁰ in 3-Position, R¹⁸ - R²⁰ = H, mit COY in 4-Position, Y = N-Ampicillino.

Substanz (29) wurde analog zu Substanz 28 aus 4- (2, 3-Diacetoxybenzoylamino) -benzoesäure (Substanz 17) und Ampicillin-Natriumsalz erhalten. Das Rohprodukt wurde mittels präparativer HPLC getrennt (Nucleosil 7 C 18, Macherey & Nagel, Laufmittel Acetonitril/Wasser 50/50 + 0,05 % Trifluoressigsäure).
¹H-NMR (DMSO-d₆, δ in ppm): 7,3 - 7,9 (m, 12 H, ArH), 5,9 (d, 1 H, α-CH), 5,53 (q, 1 H, 6-CH), 5,41 (d, 1 H, 7-CH), 4,2 (s, 1 H, 3-CH), 2,20, 2,28 (s, 2 x 3 H, CH₃), 1,40, 1,52 (s, 2 x 3H, CH₃)

### Beispiel 30

### (S)-N-[3-(2,3-Di(methoxycarbonyloxy-benzoyl)-oxazolidin-4-oyl]-ampicillin

(nach IUPAC: (S)-6-(2-([3-(2,3-Di-methoxycarbonyloxy-benzoyl) -oxazolidin-4-carbonyl]-amino)-2-phenyl-acetylamino)-3,3-dimethyl-7-oxo-4-thia-1-azabicyclo[3.2.0]-heptan-2-carbonsäure) (30)

Formel I mit R¹ = OCOOCH₃, R² = R¹⁴ in 3-Position, mit Z = O, R¹⁶, R¹⁷ = H, (S-Form), Y = N-Ampicillino

Substanz 30 wurde analog zu Substanz 28 aus (S-3-(2,3-Di-methoxycarbonyloxy-benzonyl)-oxazolidin-4-carbonsäure und Ampicillintrihydrat hergestellt.

### Beispiel 31

### N-[L-2,6-Bis- (2,3-diacetoxy-benzoylamino)-hexanoyl]-ampicillin

(nach IUPAC: 6-{L-2-[2,6-Bis-(2,3-diacetoxy-benzoylamino)-hexanyloamino]-2-phenyl-acetylamin)-3,3-dimethyl-7-oxo-4-thia-1-aza-bicyclo [3.2.0] -heptan-2-carbonsäure) (31)

Formel I mit R¹ = OCOCH₃, R² = R¹¹ in 3-Position, mit R⁶ = R¹², n = 4, R¹⁵ = 2,3-OCOCH₃ (L-Form), Y = N-Ampicillino

Substanz 31 wurde analog zu Substanz 28 aus L-2,6-Bis-(2,3-diacetoxybenzoylamino)-hexansäure (Substanz 25) und Ampicillin-Trihydrat erhalten.

### Beispiel 32

### N-[L-3-Acetoxy-2-(2, 3-diacetoxy-benzoylamino)-propionyl]-ampicillin

(nach IUPAC: 6-{L-2-[3-Acetoxy-2-(2,3-diacetoxybenzoylamino) -propionylamino]-2-phenyl-acetylamino}-3,3-dimethyl-7-oxo-4-thia-1-aza-bicyclo[3.2.0]heptan-2-carbonsäure) (32)

Formel I mit R¹ = OCOCH₃, R² = R¹¹ in 3-Position, mit R⁶ = CH₂OCOCH₃ (L-Form), Y = N-Ampicillino

Substanz 32 wurde analog zu Substanz 27 aus L-3-Acetoxy-2-(2,3-diacetoxy-benzoylamino)-propionsäure und Ampicillin-Trihydrat mit einer Reinheit des Rohproduktes nach HPLC (Nucleosil 7 C 18, Macherey & Nagel, Laufmittel Acetonitril/Wasser 40/60 + 0,05 % Trifluoressigsäure) von 85 % hergestellt.

### Beispiel 33

### N-[L-2-(2,3-Diacetoxy-benzoylamino)-propionyl]-cefadroxil

(nach IUPAC: 7-{L-2-[2-(2,3-Diacetoxy-benzoylamino)-propionylamino]-2-(4-hydroxyphenyl)-acetylamino}-3-methyl-8-oxo-5-thia-1-aza-bicyclo[4.2.0]oct-2-en-2-carbonsäure) (33)

Formel I mit R¹ = OCOCH₃, R² = R¹¹ in 3-Position, mit R⁶ = CH₃ (L-Form), Y = N-Cefadroxil (33) Substanz 33 wurde analog zu Substanz 28 aus L-2-(2,3-Diacetoxybenzoylamino)-propionsäure (Substanz 20) und Cefadroxil hergestellt. Die Reinigung erfolgte mittels präparativer HPLC (RP 18, Acetonitril/Wasser 30/70 + 0,1 % Trifluoessigsäure).

Die erhaltene Verbindung (33) wies eine Reinheit von 91 % auf.

### Beispiel 34

### N-[L-3-Benzyloxy-2-(2,3-diacetoxy-benzoylamino)propionyl]-ampicillin

Formel I mit R¹ = OCOCH₃, R² = R¹¹ in 3-Position, R⁶ = CH₂OCH₂ (C₆H₅), L-Form, Y = N-Ampicillino

Die Substanz wurde analog Substanz 28 aus L-3-Benzyloxy-2-(2,3-diacetoxy-benzoylamino)-propionsäure (Substanz 23a) und Ampicillin-Trihydrat erhalten.

### Beispiel 35

### N-[L-2,6-Bis-(2,3-diacetoxy-benzoylamino)hexanoyl]-amoxicillin

Formel I mit R¹ = OCOCH₃, R² = R¹¹ in 3-Position, R⁶ = R¹², n = 4, R¹⁵ = 2,3-OCOCH₃, L-Form, Y = N-Amoxicillino

Die Substanz wurde analog zu Substanz 28 aus L-2,6-Bis-(2,3-diacetoxy benzoylamino)-hexansäure (Substanz 25) und Amoxicillin-Trihydrat in einer Ausbeute von 84 % der Theorie erhalten.

### Beispiel 36

### N-[3,5-Bis-(2,3-diacetoxy-benzoylamino)-benzoyl]ampicillin

Formel I mit R¹ = OCOCH3, R² = R¹⁰ in 3-Position, R¹⁸ = R¹⁹ = H, R²⁰ = (2,3-Diacetoxy)-benzoylamino in 5-Position, COY in 3-Position, Y = N-Ampicillino

Die Substanz wurde analog Substanz 28 aus 3,5-Bis-(2, 3-diacetoxy-benzoylamino)-benzoesäure (Substanz 16b) und Ampicillin-Natriumsalz erhalten.

### Beispiel 37

### N-(4-[(2,3-Di-methoxycarbonyloxy-benzoyl)-methylamino]-benzoyl}-ampicillin

Formel I mit R¹ = OCOOCH₃, R² = R¹⁰ in 3-Position, R¹⁹ = CH₃, R¹⁸ = R²⁰ = H, COY in 4-Position, Y = N-Ampicillino

Zu einer Lösung von 245 mg Ampicillin-Natriumsalz in 5 ml wässrigem Tetrahydrofuran (80 % THF) wurde bei -5°C unter Rühren eine Lösung von 280 mg 4-[ (2,3-Di-methoxycarbonyloxy-benzoyl)-methyl-amino]-benzoylchlorid (hergestellt aus 4-[(2, 3-Di-methoxycarbonyloxy-benzoyl) -methyl-amino]-benzoesäure = Substanz 16a und Phosphorpentachlorid) in 3 ml absolutem Tetrahydrofuran getropft. Die Mischung rührte man je 1 h bei (0°C bzw 20°C und dampfte dann im Vakuum ein. Der Rückstand wurde mit 1 N Salzsäure auf pH 3 gebracht und mit Ethylacetat extrahiert. Die Extrakte wurden mit wässriger Natriumchloridlösung gewaschen und über Natriumsulfat getrocknet. Nach weitgehendem Einengen wurde mit Petrolether versetzt. Dabei fielen 425 mg (88 % der Theorie) der Titelverbindung in Form eines weißen Pulvers an.

### Na-Salze der Substanzen 27, 28, 30, 31, 35 und 37

Die Natriumsalze der o.g. Substanzen ließen sich nach folgender allgemeiner Arbeitsweise erhalten:

Eine Lösung von 1,1 g der Säure in 5 ml Ethylacetat wurde mit einer Lösung von 0,5 g Natrium-2-ethylhexanoat in 3 ml Ethylacetat versetzt und die Mischung mit 30 ml Petrolether (Sdp. 40 - 65°C) verdünnt. Die dabei angefallenen Natriumsalze wurden isoliert, im Vakuum getrocknet und mittels präparativer HPLC an einer RP 18-Säule gereinigt.
Ausbeuten: 50 - 80 %

## Patentansprüche

1. Catecholderivate der allgemeinen Formel I 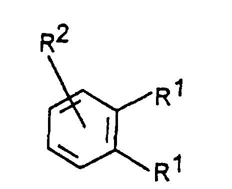 in der R¹ identisch oder unabhängig voneinander OH und/oder OAcyl bedeutet und R² in 3- und/oder 4-Stellung folgende Gruppen darstellt:
a. aromatische Azomethincarbonsäurereste und/oder Azobenzolcarbonsäurereste: 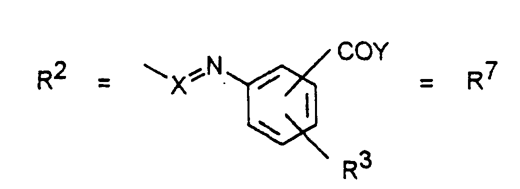
X = CH, N, CH=CH-CH
Y = OA, mit A = H, Alkyl, Aryl, Aralkyl, ein Alkalimetallion, ein Ammoniumion oder ein substituiertes Ammoniumion ist, oder
Y = ein Wirkstoffrest, der eine OH- oder NH-Gruppe enthält, ist, ausgewählt aus der Antibiotikagruppe, die die β-Lactame Cephalosporin, insbesondere Cephalexin, Cephadroxil oder Claforan, und Penicillin, insbesondere Ampicillin oder Amoxicillin, und ferner Tetracyclindeviate vom Typ des Aminodoxycyclins sowie Antibiotika vom Typ der Aminoglykoside, Makrolide, Chinolone und Carbapeneme umfaßt,
R³ = ein oder zwei OAcyl-Reste, wenn R¹ = OH oder OAcyl oder
R³ = H, wenn R¹ = OAcyl oder 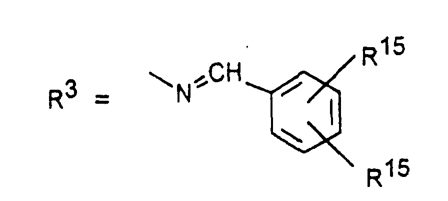
R¹⁵ identisch oder unabhängig voneinander H und/oder OAcyl bedeutet
oder 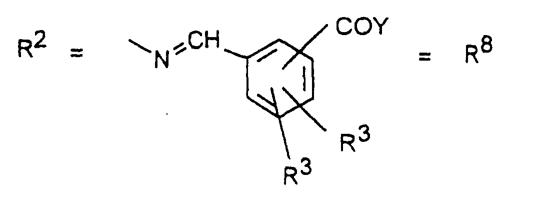
Y = OA mit A = H, Alkyl, Aryl, Aralkyl, ein Alkalimetallion, ein Ammoniumion oder ein substituiertes Ammoniumion, oder
Y = ein Wirkstoffrest, der eine NH- oder OH-Gruppe enthält, ist, wie oben definiert,
R³ identisch oder unabhängig voneinander H, OH, Oacyl bedeutet,
b. Benzhydrazonreste: 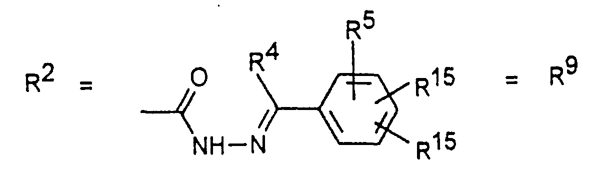
R¹⁵ identisch oder unabhängig voneinander H, OH, OAcyl bedeutet,
R⁴ und/oder R⁵ H, COY ist, wobei
Y = OA mit A = H, Alkyl, Aryl, Aralkyl, ein Alkalimetallion, ein Ammoniumion oder ein substituiertes Ammoniumion oder
Y = ein Wirkstoffrest, der eine NH- oder OH-Gruppe enthält, ist, wie oben definiert,
c. Aminobenzoesäurereste: 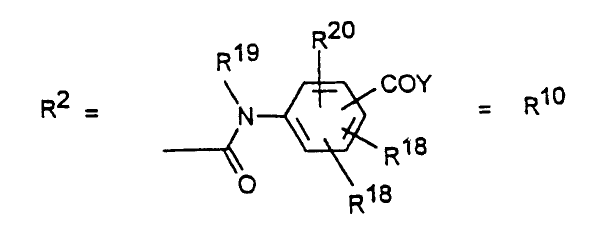
Y = OA mit A = H, Alkyl, Aryl, Aralkyl, ein Alkalimetallion, ein Ammoniumion oder ein substituiertes Ammoniumion, oder
Y = ein Wirkstoffrest, der eine NH- oder OH-Gruppe enthält, ist, wie oben definiert,
R¹⁹ = H, Alkyl
R²⁰ = H, Alkyl, Halogen, OH, OAlkyl, OAcyl oder 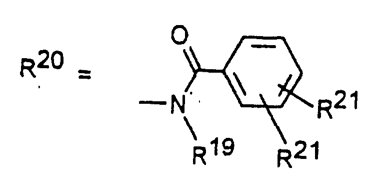
R¹⁸ und R²¹ jeweils identisch oder unabhängig voneinander H, OH, OAcyl, OAlkyl in 2,3- und/oder 3,4-Position bedeuten,
d. Aminosäurereste: 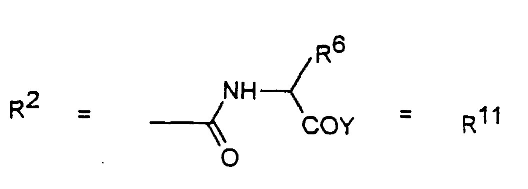
Y = OA mit A = H, Alkyl, Aryl, Aralkyl, ein Alkalimetallion, ein Ammoniumion oder ein substituiertes Ammoniumion, oder
Y = ein Wirkstoffrest, der eine NH- oder OH-Gruppe enthält, ist, wie oben definiert,
R⁶ = Alkyl, Hydroxyalkyl (mit C₁ - C₅, wenn R¹ = OAcyl, und C₃ - C₅, wenn R¹ = OH), oder Alkoxyalkyl, Acyloxyalkyl, Arylalkoxyalkyl, 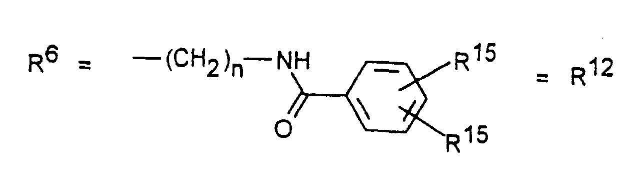
R¹⁵ identisch oder unabhängig voneinander H, OH, Oacyl bedeutet,
n eine ganze Zahl zwischen 1 und 5 ist, wenn R¹ Oacyl und R¹⁵ H und/oder Oacyl ist oder
n eine ganze Zahl zwischen 1 und3 ist, wenn R¹ OH und R¹⁵ H und/oder OH ist, oder 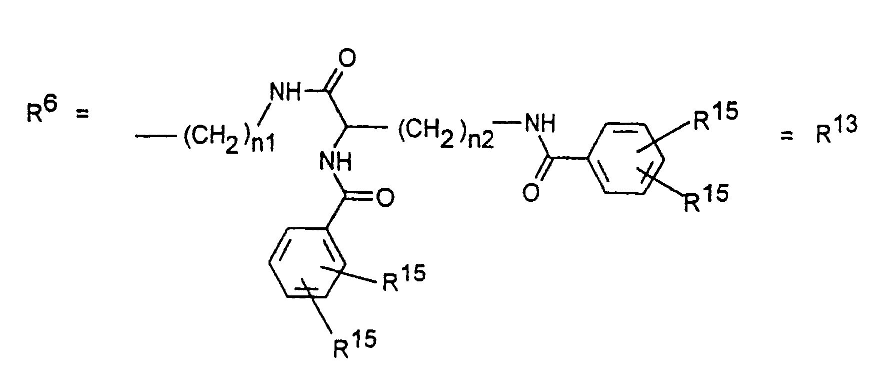
R¹⁵ identisch oder unabhängig voneinander H, OH, OAcyl bedeutet, n₁ und n₂ eine ganze Zahl zwischen 1 und 5 ist,
e. Pyrrolidin- und/oder Oxazolidincarbonsäurereste: 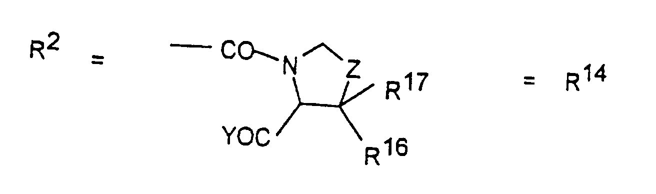
Z = O, CH₂
R¹⁶ und R¹⁷ identisch oder unabhängig voneinander H, Alkyl oder Aryl bedeuten,
Y = OA mit A = H, Alkyl, Aryl, Aralkyl, ein Alkalimetallion, ein Ammonium oder ein substituiertes Ammoniumion, oder
Y = ein Wirkstoffrest, der eine NH- oder OH-Gruppe enthält, ist, wie oben definiert,
f. Formyl-O-carboxymethyloxime:
R² = CH=NOCH₂COY,
Y = OA, mit A = H, Alkyl, Aryl, Aralkyl, ein Alkalimetallion, ein Anunoniumion oder ein substituiertes Acurzoniumion, oder
Y = ein Wirkstoffrest, der eine NH- oder OH-Gruppe enthält, ist, wie oben definiert.

2. Verbindungen der Formel I gemäß Anspruch 1, wobei R² = R¹¹ mit Y = OH und R⁶ = C₁-C₄-Alkyl bedeuten.

3. Verbindungen der Formel I gemäß Anspruch 1, wobei R² = R¹¹ mit Y = OH, R⁶ = R¹² mit R¹⁵ = OAcyl und n = 1 - 5 bedeuten.

4. Verbindungen der Formel I gemäß Anspruch 1, wobei R² = R¹¹ mit Y = OH, R⁶ = R¹³ mit R¹⁵ = OAcyl und n₁ und n₂ eine ganze Zahl zwischen 1 und 4 bedeuten.

5. Verbindungen der allgemeinen Formel I gemäß Anspruch 1, dadurch gekennzeichnet, daß die Verbindung 2-(2,3-Diacetoxybenzoylamino)-benzoesäure ist.

6. Verbindungen der allgemeinen Formel I gemäß Anspruch 1, dadurch gekennzeichnet, daß die Verbindung L-2-(2,3-Diacetoxybenzoylamino) -propionsäure ist

7. Verbindungen der allgemeinen Formel I gemäß Anspruch 1, dadurch gekennzeichnet, daß die Verbindung L-2,6-Bis-(2,3-diacetoxybenzoylamino)-hexansäure ist.

8. Verbindungen der Formel I gemäß Anspruch 1, wobei Y der Rest eines Cephalosporins ist.

9. Verbindungen der Formel I gemäß Anspruch 1, wobei Y der Rest eines Penicillins ist.

10. Verbindungen der Formel I gemäß Anspruch 1, wobei Y ein Ampicillinrest ist.

11. Verbindungen der Formel I gemäß Anspruch 1, wobei Y ein Amoxicillinrest ist.

12. Verbindungen der Formel I gemäß Anspruch 1, wobei Y ein Tetracyclinrest mit NH- oder OH-Gruppe ist.

13. Verbindungen der Formel I gemäß Anspruch 1, wobei Y der Rest eines Makrolid mit NH- oder OH-Gruppe ist.

14. Verbindungen der Formel I gemäß Anspruch 1, wobei Y der Rest eines Chinolons mit NH- oder OH-Gruppe ist.

15. Verbindungen der Formel I gemäß Anspruch 1, wobei Y der Rest eines Carbapenems mit NH- oder OH-Gruppe ist.

16. Verbindungen der allgemeinen Formel I gemäß Anspruch 1, dadurch gekennzeichnet, daß die Verbindung N-[L-2-(2,3-Diacetoxybenzoylamino)-propionyl]-ampicillin ist.

17. Verbindungen der allgemeinen Formel I gemäß Anspruch 1, dadurch gekennzeichnet, daß die Verbindung N-[2-(2,3-Diacetoxybenzoylamino)-benzoyl]-ampicillin ist.

18. Verbindungen der allgemeinen Formel I gemäß Anspruch 1, dadurch gekennzeichnet, daß die Verbindung N-[4-(2,3-Diacetoxybenzoylamino)-benzoyl]-ampicillin ist.

19. Verbindungen der allgemeinen Formel I gemäß Anspruch 1, dadurch gekennzeichnet, daß die Verbindung (S)-N-[3-(2,3-Dimethoxycarbonyloxybenzoyl)-oxazolidin-4-oyl]-ampicillin ist.

20. Verbindungen der allgemeinen Formel I gemäß Anspruch 1, dadurch gekennzeichnet, daß die Verbindung N-[L-3-Acetoxy-2-(2,3-diacetoxy-benzoylamino)-propionyl]-ampicillin ist.

21. Verwendung der Verbindungen der Formel I nach Anspruch 1 zur Herstellung eins Therapeutikums zur Bekämpfung von bakteriellen Infektionen.

22. Verwendung der Verbindungen der Formel I nach Anspruch 1 zur Herstellung von Therapeutika zur Bekämpfung von Erkrankungen, die auf eine Störung des Eisenstoffwechsels zurückzuführen sind.

23. Arzneimittel enthaltend eine Verbindung der Formel I nach Anspruch 1 zusammen mit üblichen Trägermaterialien.

## Claims

1. Catechol derivatives of the general formula I 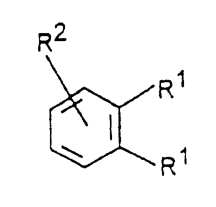 in which in which R¹ identically or mutually independently mean OH and/or Oacyl and R² represents the following groups in position 3 and/or 4:
a. aromatic azcmethinecarboxylic acid residues and/or azobenzenecarboxylic acid residues: 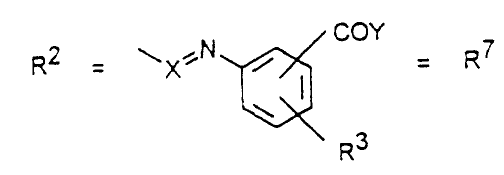
X = CH, N, CH=CH-CH
Y = OA, where A is H, alkyl, aryl, aralkyl, an alkali metal ion, an ammonium ion or a substituted ammonium ion, or
Y = an active substance residue, which contains an OH or NH group, selected from the group of antibiotics comprising the β-lactams cephalosporin, in particular cephalexin, cephadroxil or Claforan, and penicillin, particular ampicillin or amoxicillin, and furthermore tetracycline derivatives of the aminodoxycycline type as well as antibiotics of the aminoglycoside, macrolide, quinolone and carbapenem type,
R³ = one or two Oacyl residues, if R¹ = OH or Oacyl or
R³ = H, if R¹ = Oacyl or 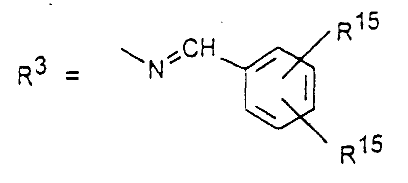
R¹⁵ = identically or mutually independently mean H, Oacyl
or 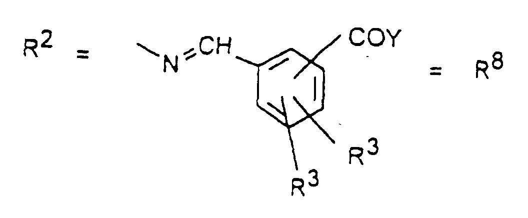
Y = OA, where A = H, alkyl, aryl, aralkyl, an alkali metal ion, an ammonium ion or a substituted ammonium ion, or
Y = is an active substance residue, which contains an NH or OH group, as defined above,
R³ = identically or mutually independently mean H, OH, Oacyl,
b. benzhydrazone residues: 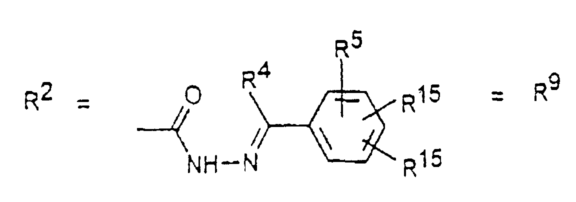
R¹⁵ identically or mutually independently mean H, OH, Oacyl,
R⁴ and/or R⁵ is H, COY, wherein
Y = OA, where A = H, alkyl, aryl, aralkyl, an alkali metal ion, an ammonium ion or a substituted ammonium ion, or
Y = is an active substance residue, which contains an NH or OH group, as defined above,
c. aminobenzoic acid residues: 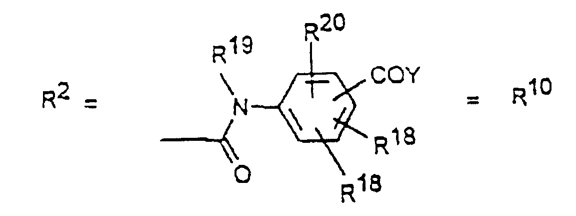
Y = OA, where A = H, alkyl, aryl, aralkyl, an alkali metal ion, an ammonium ion or a substituted ammonium ion, or
Y = is an active substance residue, which contains an NH or OH group, as defined above,
R¹⁹ = H, alkyl
R²⁰ = H, alkyl, halogen, OH, Calkyl, Oacyl or 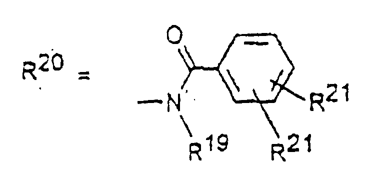
R¹⁸ and R²¹ each identically or mutually independently mean H, OH, Oacyl, Oalkyl in position 2,3 and/or 3,4,
d. amino acid residues: 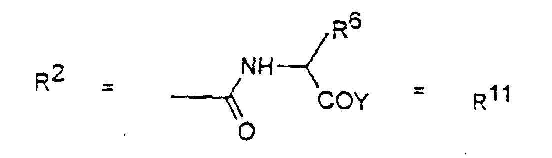
Y = OA, where A = H, alkyl, aryl, aralkyl, an alkali metal ion, an ammonium ion or a substituted ammonium ion, or
Y = is an active substance residue, which contains an NH or OH group, as defined above,
R⁶ = alkyl, hydroxyalkyl (C₁-C₅ if R¹ = Oacyl, and C₃-C₅ if R¹ = OH), alkoxyalkyl, acyloxyalkyl, arylalkoxyalkyl, 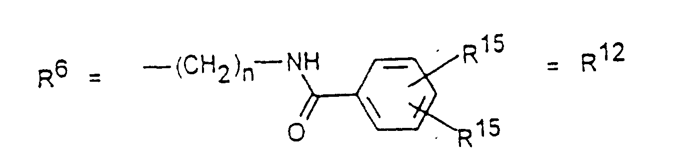
R¹⁵ identically or mutually independently mean H, OH, Oacyl,
n means an integer between 1 and 5, if R¹ is Oacyl and R¹⁵ is H and/or Oacyl or
n means an integer between 1 and 3, if R¹ is OH and R¹⁵ is H and/or OH, or 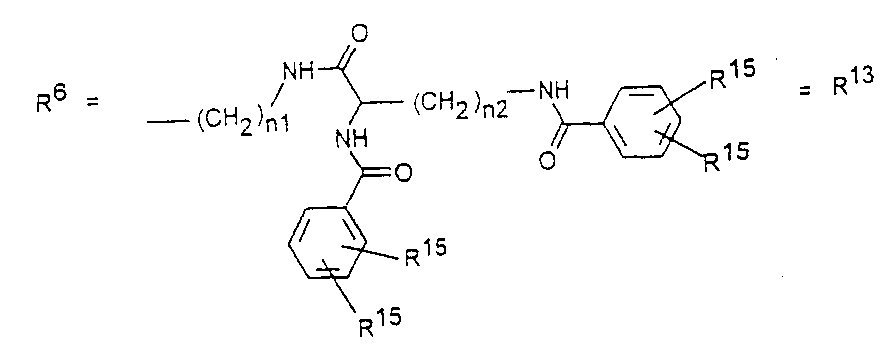
R¹⁵ identically or mutually independently mean H, OH, Oacyl, n₁ and n₂ are an integer between 1 and 5,
e. pyrrolidine- and/or oxazolidinecarboxylic acid residues: 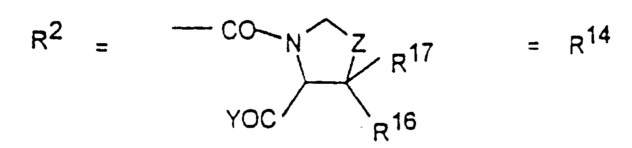
Z = O, CH₂,
R¹⁶ and R¹⁷ identically or mutually independently mean H, alkyl or aryl,
Y = OA, where A = H, alkyl, aryl, aralkyl, an alkali metal ion, ammonium or a substituted ammonium ion, or
Y = is an active substance residue, which contains an NH or OH group, as defined above,
f. formyl-O-carboxymethyl oximes:
R² = CH=NOCH₂COY,
Y = OA, where A = H, alkyl, aryl, aralkyl, an alkali metal ion, an ammonium ion or a substituted ammonium ion, or
Y = is an active substance residue, which contains an NH or OH group, as defined above.

2. Compounds of the formula I according to claim 1, wherein R² = R¹¹ where Y = OH and R⁶ = C₁-C₄ alkyl.

3. Compounds of the formula I according to claim 1, wherein R² = R¹¹ where Y = OH, R⁶ = R¹² where R¹⁵ = Oacyl and n = 1-5.

4. Compounds of the formula I according to claim 1, wherein R² = R¹¹ where Y = OH, R⁶ = R¹³ where R¹⁵ = Oacyl and n₁ and n₂ mean an integer between 1 and 4.

5. Compounds of the general formula I according to claim 1, characterised in that the compound is 2-(2,3-diacetoxybenzoylamino)benzoic acid.

6. Compounds of the general formula I according to claim 1, characterised in that the compound is L-2-(2,3-diacetoxybenzoylamino)propionic acid.

7. Compounds of the general formula I according to claim 1, characterised in chat the compound is L-2,6-bis(2,3-diacetoxybenzoylamino)hexanoic acid.

8. Compounds of the formula I according to claim 1, wherein Y is the residue of a cephalosporin.

9. Compounds of the formula I according to claim 1, wherein Y is the residue of a penicillin.

10. Compounds of the formula I according to claim 1, wherein Y is an ampicillin residue.

11. Compounds of the formula I according to claim 1, wherein Y is an amoxicillin residue.

12. Compounds of the formula I according to claim 1, wherein Y is a tetracycline residue with an NH or OH group.

13. Compounds of the formula I according to claim 1, wherein Y is the residue of a macrolide with an NH or OH group.

14. Compounds of the formula I according to claim 1, wherein Y is the residue of a quinolone with an NH or OH group.

15. Compounds of the formula I according to claim 1, wherein Y is the residue of a carbapenem with an NH or OH group.

16. Compounds of the general formula I according to claim 1, characterised in that the compound is N-[L-2-(2,3-diacetoxybenzoylamino)propionyl]ampicillin.

17. Compounds of the general formula I according to claim 1, characterised in that the compound is N-[2-(2,3-diacetoxybenzoylamino)benzoyl]ampicillin.

18. Compounds of the general formula I according to claim 1, characterised in that the compound is N-[4-(2,3-diacetoxybenzoylamino)benzoyl]ampicillin.

19. Compounds of the general formula I according to claim 1, characterised in that the compound is (S)-N-[3-(2,3-dimethoxycarbonyloxybenzoyl)oxazolidin-4-oyl]-ampicillin.

20. Compounds of the general formula I according to claim 1, characterised in that the compound is N-[L-3-acetoxy-2-(2,3-diacetoxybenzoylamino)propionyl]-ampicillin.

21. Use of the compounds of the formula I according to claim 1 for the production of a therapeutic preparation for combating bacterial infections.

22. Use of the compounds of the formula I according to claim 1 for the production of therapeutic preparations for combating diseases attributable to disruption of the iron metabolism.

23. Pharmaceutical preparations containing a compound of the formula I according to claim 1 together with conventional excipients.

## Revendications

1. Dérivés de catéchol de formule générale I 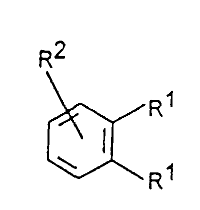 dans laquelle les groupes R¹ sont identiques ou représentent indépendamment l'un de l'autre un groupe OH et/ou un groupe Oacyle et R² représente les groupes suivants en position 3 et/ou en position 4 :
a. des restes aromatiques d'acide azométhine-carboxylique et/ou d'acide azobenzène-carboxylique 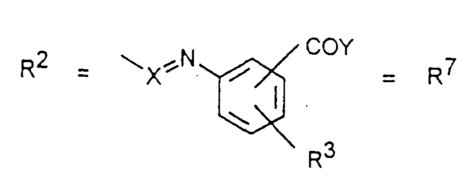
X représente CH, N, CH=CH-CH
Y représente OA, avec A = H, alkyle, aryle, aralkyle, un ion de métal alcalin, un ion ammonium ou un ion ammonium substitué, ou bien
Y représente un reste de substance active qui contient un groupe OH ou NH, choisi dans le groupe des antibiotiques qui comprend les β-lactames céphalosporine, notamment céphalexine, céphadroxil ou claforan, et pénicilline, en particulier ampicilline ou amoxicilline et en outre des dérivés de tétracycline du type de l'aminodoxycycline, ainsi que des antibiotiques du type des aminoglycosides, des macrolides, des quinolones et des cabapénèmes,
R³ représente un ou deux restes Oacyle lorsque R¹ est un groupe OH ou Oacyle ou bien
R³ représente H lorsque R¹ est un groupe Oacyle, ou bien 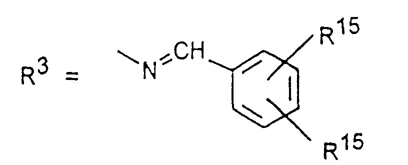
les groupes R¹⁵ sont identiques ou représentent indépendamment l'un de l'autre H et/ou Oacyle
ou bien 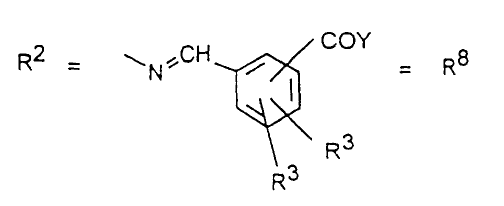
Y représente un groupe OA avec A = H, alkyle, aryle, aralkyle, un ion de métal alcalin, un ion ammonium ou un ion ammonium substitué, ou bien
Y est un reste de substance active qui contient un groupe NH ou OH, comme défini ci-dessus
les groupes R³ sont identiques ou représentent indépendamment l'un de l'autre H, OH, Oacyle,
b. des restes de benzhydrazone : 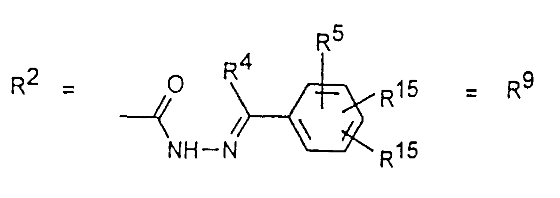 les groupes R¹⁵ sont identiques ou représentent indépendamment l'un de l'autre H, OH, Oacyle,
R⁴ et/ou R⁵ représentent H, un groupe COY, où
Y est un groupe OA avec A = H, alkyle, aryle, aralkyle, un ion de métal alcalin, un ion ammonium ou un ion ammonium substitué, ou bien
Y est un reste de substance active qui contient un groupe NH ou OH, comme défini ci-dessus,
c. des restes d'acide amino-benzoïque : 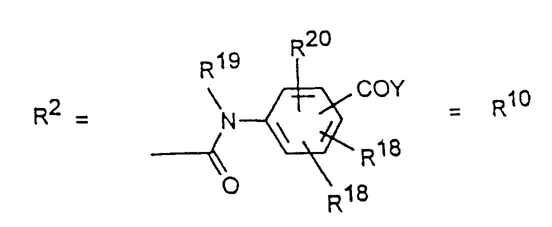
Y est un groupe OA avec A = H, alkyle, aryle, aralkyle, un ion de métal alcalin, un ion ammonium ou un ion ammonium substitué, ou bien
Y est un reste de substance active qui contient un groupe NH ou OH, comme défini ci-dessus
R¹⁹ représente H, un groupe alkyle
R²⁰ représente H, un groupe alkyle, un halogène, un groupe OH, Oalkyle, Oacyle, ou bien 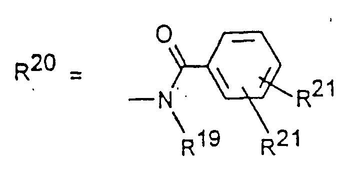
les groupes R¹⁸ et les groupes R²¹ sont dans chaque cas identiques ou représentent indépendamment l'un de l'autre H, OH, Oacyle, Oalkyle, en positions 2,3 ou 3,4,
d. des restes d'amino-acides : 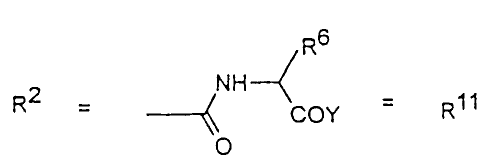
Y représente un groupe OA avec A = H, alkyle, aryle, aralkyle, un ion de métal alcalin, un ion ammonium ou un ion ammonium substitué, ou bien
Y est un reste de substance active qui contient un groupe NH ou OH, comme défini ci-dessus,
R⁶ est un groupe alkyle, hydroxyalkyle (avec 1 à 5 atomes de carbone lorsque R¹ est un groupe Oacyle et avec 3 à 5 atomes de carbone lorsque R¹ est un groupe OH), ou alkoxyalkyle, acyloxyalkyle, arylalkoxyalkyle, ou bien 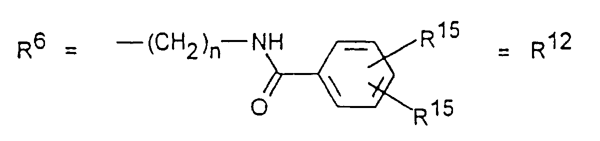
les groupes R¹⁵ sont identiques ou - représentent, indépendamment l'un de l'autre, H, OH, Oacyle
n est un nombre entier de 1 à 5 lorsque R¹ est un groupe Oacyle et R¹⁵ représente H et/ou un groupe Oacyle, ou bien
n est un nombre entier de 1 à 3 lorsque R¹ est un groupe OH et R¹⁵ représente H et/ou un groupe OH, ou bien 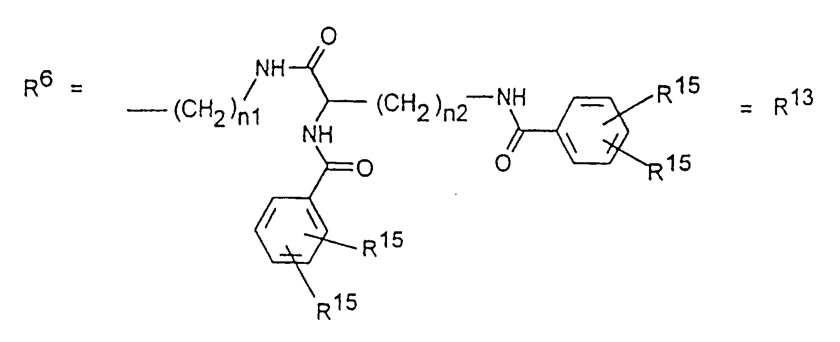 les groupes R¹⁵ sont identiques ou représentent indépendamment l'un de l'autre H, OH, Oacyle, n₁ et n₂ représentent un nombre entier de 1 à 5,
e. des restes d'acides pyrrolidine- et/ou oxazolidine-carboxyliques : 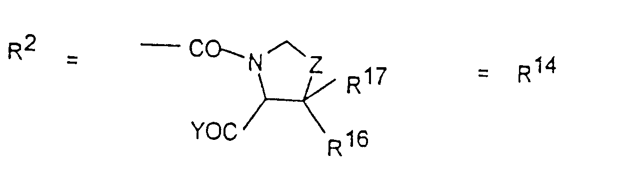
Z représente O, CH₂
R¹⁶ et R¹⁷ sont identiques ou représentent indépendamment l'un de l'autre H, alkyle ou aryle
Y est un groupe OA avec A = H, alkyle, aryle, aralkyle, un ion de métal alcalin, un ion ammonium ou un ion ammonium substitué , ou bien
Y est un reste de substance active qui contient un groupe NH ou un groupe OH comme défini ci-dessus,
f. des formyl-O-carboxyméthyloximes :
R² = CH=NOCH₂COY, où
Y représente un groupe OA avec A = H, alkyle, aryle, aralkyle, un ion de métal alcalin, un ion ammonium ou un ion ammonium substitué, ou bien
Y est un reste de substance active qui contient un groupe NH ou un groupe OH comme défini ci-dessus.

2. Composés de formule I suivant la revendication 1, dans lesquels R² est égal à R¹¹ avec Y = OH et R⁶ est un groupe alkyle en C₁ à C₄.

3. Composés de formule I suivant la revendication 1, dans lesquels R² est égal à R¹¹ avec Y = OH, R⁶ est égal à R¹² avec R¹⁵ = Oacyle et n a une valeur de 1 à 5.

4. Composés de formule I suivant la revendication 1, dans lesquels R² est égal à R¹¹ avec Y = OH, R⁶ est égal à R¹³ avec R¹⁵ = Oacyle et n₁ et n₂ représentent un nombre entier de 1 à 4.

5. Composés de formule générale I suivant la revendication 1, caractérisés en ce que le composé est l'acide 2-(2,3-diacétoxybenzoylamino)-benzoïque.

6. Composés de formule générale I suivant la revendication 1, caractérisés en ce que le composé est l'acide L-2-(2,3-diacétoxybenzoylamino)-propionique.

7. Composés de formule générale I suivant la revendication 1, caractérisés en ce que le composé est l'acide L-2,6-bis-(2,3-diacétoxybenzoylamino)-hexanoïque.

8. Composés de formule I suivant la revendication 1, dans lesquels Y est le reste d'une céphalosporine.

9. Composés de formule I suivant la revendication 1, dans lesquels Y est le reste d'une pénicilline.

10. Composés de formule I suivant la revendication 1, dans lesquels Y est un reste d'ampicilline.

11. Composés de formule I suivant la revendication 1, dans lesquels Y est un reste d'amoxicilline.

12. Composés de formule I suivant la revendication 1, dans lesquels Y est un reste de tétracycline portant un groupe NH ou OH.

13. Composés de formule I suivant la revendication 1, dans lesquels Y est le reste d'un macrolide portant un groupe NH ou OH.

14. Composés de formule I suivant la revendication 1, dans lesquels Y est le reste d'une quinolone portant un groupe NH ou OH.

15. Composés de formule I suivant la revendication 1, dans lesquels Y est le reste d'un carbapénème portant un groupe NH ou OH.

16. Composés de formule générale I suivant la revendication 1, caractérisés en ce que le composé est la N-[L-2-(2,3-diacétoxybenzoylamino)-propionyl] -ampicilline.

17. Composés de formule générale I suivant la revendication 1, caractérisés en ce que le composé est la N-[2-(2,3-diacétoxybenzoylamino)-benzoyl]-ampicilline.

18. Composés de formule générale I suivant la revendication 1, caractérisés en ce que le composé est la N-[4-(2,3-diacétoxybenzoylamino)-benzoyl]-ampicilline.

19. Composés de formule générale I suivant la revendication 1, caractérisés en ce que le composé est la (S)-N-[3-(2,3-diméthoxycarbonyloxy-benzoyl)-oxazolidine-4-oyl]-ampicilline.

20. Composés de formule générale I suivant la revendication 1, caractérisés en ce que le composé est la N-[L-3-acétoxy-2-(2,3-diacétoxybenzoyl-amino)-propionyl]-ampicilline.

21. Utilisation des composés de formule I suivant la revendication 1 pour la préparation d'un agent thérapeutique destiné à combattre des infections bactériennes.

22. Utilisation des composés de formule I suivant la revendication 1 pour la préparation d'agents thérapeutiques destinés à combattre des maladies qui doivent être attribuées à un trouble du métabolisme du fer.

23. Médicaments contenant un composé de formule I suivant la revendication 1 en association avec des supports classiques.
